# EUROPEAN PATENT SPECIFICATION

(11) **EP 3 614 860 B1**
(45) Date of publication and mention of the grant of the patent: **02.07.2025**
(21) Application number: 18790170.7
(22) Date of filing: 25.04.2018
(51) Int. Cl.: A23L 2/60, A23L 27/00, A23L 27/30, C07G 3/00, C07H 15/24, C07H 15/256

(54) **SWEETNESS AND TASTE IMPROVEMENT OF STEVIOL GLYCOSIDE AND MOGROSIDE SWEETENERS WITH DIHYDROCHALCONES**
SÜSSE- UND GESCHMACKVERBESSERUNG VON STEVIOLGLYCOSID- ODER MOGROSIDSÜSSSTOFFEN MIT DIHYDROCHALCONEN
AMÉLIORATION DE LA SUCROSITÉ ET DU GOÛT D'ÉDULCORANTS À BASE DE GLYCOSIDE DE STÉVIOL ET DE MOGROSIDE AVEC DES DIHYDROCHALCONES

(30) Priority: 25.04.2017 US 201762489845 P
(43) Date of publication of application: 04.03.2020
(73) Proprietor: The Coca-Cola Company, Atlanta, GA 30313 (US)
(72) Inventor: PRAKASH, Indra, Alpharetta, GA 30022 (US); MA, Gil, Atlanta, GA 30308 (US); TAN, Xiaoliang, Marietta, GA 30068 (US); HIGIRO, Juvenal, Atlanta, GA 30345 (US); MERCOGLIANO, Christopher, Atlanta GA 30313 (US)
(74) Representative: Dehns
(86) International application number: PCT/US2018/029343
(87) International publication number: WO 2018/200663

(56) References cited:
- WO-A1-2013/074811
- WO-A1-2018/001703
- CN-A- 104 705 618
- JP-A- S5 122 862
- US-A- 3 739 064
- US-A- 3 751 270
- US-A1- 2008 226 799
- US-A1- 2010 233 102
- US-A1- 2011 189 108
- US-A1- 2012 183 648
- US-A1- 2015 017 284

## Description

### FIELD OF THE INVENTION

The present invention relates generally to compositions and consumables containing certain steviol glycoside and/or mogroside sweeteners and at least one dihydrochalcone.

### BACKGROUND OF THE INVENTION

Natural caloric sugars, such as sucrose, fructose and glucose, are used to provide a pleasant taste to beverages, foods, pharmaceuticals, and oral hygienic/cosmetic products. Sucrose, in particular, imparts a taste preferred by consumers. Although sucrose provides superior sweetness characteristics, it is disadvantageously caloric.

Consumers increasingly prefer non-caloric or low caloric sweeteners have been introduced to satisfy consumer demand. However, non- and low caloric sweeteners differ from natural caloric sugars in ways that frustrate consumers. On a taste basis, non-caloric or low caloric sweeteners exhibit a temporal profile, maximal response, flavor profile, mouth feel, and/or adaptation behavior that differ from sugar. Specifically, non-caloric or low caloric sweeteners exhibit delayed sweetness onset, lingering sweet aftertaste, bitter taste, metallic taste, astringent taste, cooling taste and/or licorice-like taste. On a source basis, many non-caloric or low caloric sweeteners are synthetic chemicals. Consumer desire remains high for natural non-caloric or low caloric sweeteners that tastes like sucrose.

*Stevia rebaudiana* Bertoni is a perennial shrub of the *Asteraceae* (*Compositae*) family native to certain regions of South America. Its leaves have been used for hundreds of years in Paraguay and Brazil to sweeten local teas and medicines. The plant is commercially cultivated in Japan, Singapore, Taiwan, Malaysia, South Korea, China, Israel, India, Brazil, Australia and Paraguay.

The leaves of the plant contain a mixture containing diterpene glycosides in an amount ranging from about 10% to 15% of the total dry weight. These diterpene glycosides are about 30 to 450 times sweeter than sugar. Structurally, the diterpene glycosides are characterized by a single base, steviol, and differ by the presence of carbohydrate residues at positions C13 and C19. Typically, on a dry weight basis, the four major steviol glycosides found in the leaves of Stevia are dulcoside A (0.3%), rebaudioside C (0.6-1.0%), rebaudioside A (3.8%) and stevioside (9.1%). Other glycosides identified in Stevia extract include rebaudioside B, D, E, and F, steviolbioside and rubusoside. Among these, only stevioside and rebaudioside A are available on a commercial scale.

Mogrosides are derived from *Luo Han Guo,* the common name for the sweet extract made from the fruit of Siraitia grosvenorii, a herbaceous perennial vine of the *Cucurbitaceae* family native to Southern China and Northern Thailand. *Luo Han Guo* extracts are nearly 250 times sweeter than sugar and non-caloric. The sweetness of *Luo Han Guo* is generally attributed to mogrosides.

Use of steviol glycosides and mogrosides has been limited to date by certain undesirable taste properties, including licorice taste, bitterness, astringency, sweet aftertaste, bitter aftertaste and licorice aftertaste, which become more prominent at increased concentrations and impart a taste distinct from sucrose to consumables (e.g., beverages) to which they are added. In addition, maximal sweetness of most steviol glycoside and mogrosides is generally less than what is acceptable for traditional beverage formulations in sweetened consumables (e.g., beverages).

Accordingly, there remains a need for alternative sweetener systems that provide enhanced sweetness and taste properties more like sucrose-sweetened beverages.

WO 2013/074811 discloses taste modifiers which can be used in foods, beverages, and pharmaceutical applications, and the use of taste modifiers to alleviate negative flavor attributes.

### SUMMARY OF THE INVENTION

The present invention generally relates to dihydrochalcone compounds useful for enhancing the sweetness and/or modulating the flavor of sweetened consumables, such as beverages.
The present invention provides a beverage comprising at least one sweetener and at least one dihydrochalcone, wherein the dihydrochalcone is selected from hesperetin dihydrochalcone and hesperetin dihydrochalcone-4'-β-D-glucoside, and wherein the sweetener comprises Rebaudioside M in a sweetening amount or at least one of siamenoside I and the 1,6-α isomer of siamenoside I in a sweetening amount.

In one embodiment, the dihydrochalcone is present in an amount at or below its sweetness recognition threshold concentration.

In a particular embodiment, the sweetener comprises rebaudioside M in a sweetening amount. In another particular embodiment, the sweetener comprises siamenoside I in a sweetening amount. In still another embodiment, the sweetener comprises the 1,6-α isomer of siamenoside I in a sweetening amount.

In one embodiment, the at least one dihydrochalcone is present in the beverage in an amount effective to enhance the sucrose equivalence (SE) of the beverage by at least about 1.2-fold compared to the SE of the beverage in the absence of the at least one dihydrochalcone.

In another embodiment, the at least one dihydrochalcone is present in the beverage in an amount effective to modulate one or more taste attributes of the sweetener making the consumable taste more like a sucrose-sweetened beverage compared to the beverage in the absence of the at least one dihydrochalcone.

In one embodiment, the at least one dihydrochalcone is present in the beverage in a concentration from about 1 ppm to about 50 ppm.

In one embodiment, the sweetener is present in the beverage in a concentration from about 50 ppm to about 600 ppm.

The beverage can be a full-calorie, mid-calorie, low-calorie or zero-calorie beverage.

Further described herein but not constituting an embodiment of the claimed invention is a method of enhancing the sweetness of a consumable comprises (i) providing a consumable comprising a sweetener described herein and (ii) adding at least one dihydrochalcone of the formulae detailed herein to the consumable. In exemplary embodiments, the SE of the consumable comprising the at least one dihydrochalcone is enhanced by at least about 1.2-fold compared to the SE of the consumable in the absence of the at least one dihydrochalcone.

Further described herein but not constituting an embodiment of the claimed invention is a method of enhancing the sweetness of a consumable comprises (i) providing a consumable matrix and (ii) adding at least one sweetener and at least one dihydrochalcone of the formulae detailed herein to the consumable matrix to provide a consumable. In exemplary embodiments, the at least one dihydrochalcone is added to the consumable matrix in an amount effective to enhance the SE of the consumable by at least about 1.2-fold compared to the SE of the consumable in the absence of the at least one dihydrochalcone.

Further described herein but not constituting an embodiment of the claimed invention is a method of making a consumable taste more like a sucrose-sweetened consumable comprises (i) providing a consumable comprising at least one sweetener described hereinabove and (ii) adding at least one dihydrochalcone of the formulae detailed herein to the consumable in an amount effective to modulate one or more taste attributes of the sweetener to make the consumable taste more like a sucrose-sweetened consumable when compared to the consumable in the absence of the at least one dihydrochalcone.

Further described herein but not constituting an embodiment of the claimed invention is a method of making a consumable taste more like a sucrose-sweetened consumable comprises (i) providing a consumable matrix and (ii) adding at least one sweetener and at least one dihydrochalcone of the formulae detailed herein to the consumable matrix to provide a consumable. In exemplary embodiments, the at least one dihydrochalcone is added to the consumable matrix in an amount effective to modulate one or more taste attributes of the sweetener to make the consumable taste more like a sucrose-sweetened consumable when compared to a consumable in the absence of the at least one dihydrochalcone.

### BRIEF DESCRIPTION OF THE DRAWINGS

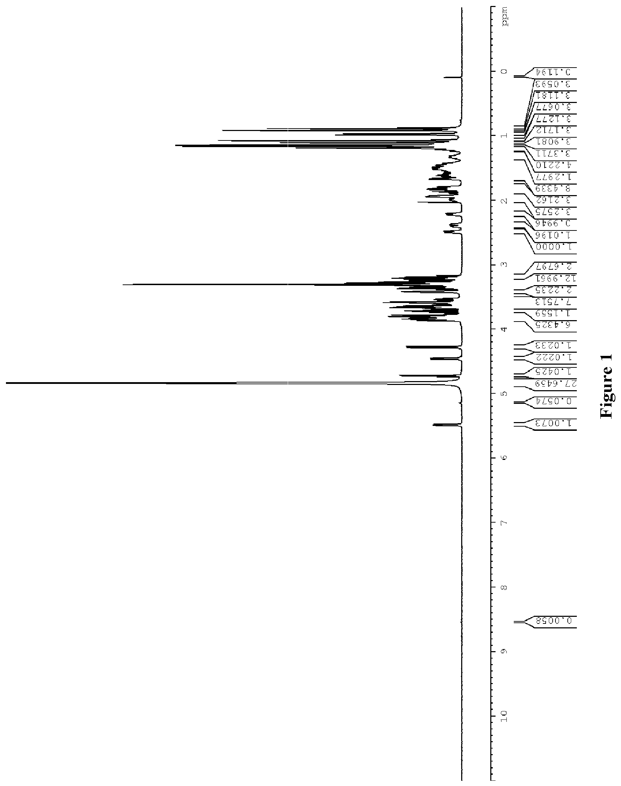

**FIG. 1** shows the ¹H NMR spectrum (500 MHz, 296K) of the 1,6-α isomer of Siamenoside I (mogro-3-O-[β-D-glucopyranoside]-24-O-{[β-D-glucopyranosyl-(1→2)]-[α-D-glucopyranosyl-(1→6)]-β-D-glucopyranoside}) in CD₃OD.

### DETAILED DESCRIPTION OF THE INVENTION

The present invention is based on the surprising finding that certain dihydrochalcones can also enhance the sweetness of certain steviol glycoside and mogroside sweeteners, and moreover, can optionally improve the sweetness profile of the steviol glycoside or mogroside sweetener.

The present invention generally relates to dihydrochalcone compounds useful for enhancing the sweetness and/or modulating the taste of certain sweeteners, particularly sweeteners in consumables such as beverages. The present invention also generally relates to methods of enhancing the sweetness and/or modulating the taste of certain sweeteners in consumables, such as beverages, using dihydrochalcone compounds.

### I. Definitions

"Alkyl", as used herein, generally refers to a noncyclic, cyclic, linear or branched, unsaturated or saturated hydrocarbon such as those containing from 1 to 22 carbon atoms, and specifically includes methyl, ethyl, propyl, isopropyl, cyclopropyl, butyl, isobutyl, t-butyl, pentyl, cyclopentyl, isopentyl, neopentyl, hexyl, isohexyl, cyclohexyl, cyclohexylmethyl, 3-methylpentyl, 2,2-dimethylbutyl, and 2,3-dimethylbutyl. The term includes both substituted and unsubstituted alkyl groups. Alkyl groups can be optionally substituted with one or more moieties selected from, for example, hydroxyl, amino, halo, deutero, alkylamino, arylamino, alkoxy, aryloxy, nitro, cyano, sulfonic acid, sulfate, phosphonic acid, phosphate, or phosphonate, or any other viable functional group, either unprotected, or protected, as necessary, as known to those skilled in the art, for example, as taught in T. W. Greene and P. G. M. Wuts, "Protective Groups in Organic Synthesis," 3ed., John Wiley & Sons, 1999.

The term "lower alkyl," as used herein, and unless otherwise specified, refers to a C1 to C5 saturated straight, branched, or if appropriate, a cyclic (for example, cyclopropyl) alkyl group, including both substituted and unsubstituted forms. Unless otherwise specifically stated in this application, when alkyl is a suitable moiety, lower alkyl is preferred.

"Consumables," as used herein, mean substances which are contacted with the mouth of man or animal, including substances which are taken into and subsequently ejected from the mouth and substances which are drunk, eaten, swallowed or otherwise ingested, and are safe for human or animal consumption when used in a generally acceptable range.

"Sweetness enhancer", as used herein, refers to a compound that enhances, amplifies or potentiates the perception of sweetness of a consumable (e.g. a beverage) when said compound is present in the consumable in a concentration at or below the compound's sweetener recognition threshold, i.e. a concentration at which the compound does not contribute any noticeable sweet taste in the absence of additional sweetener(s). The term "sweetness recognition threshold concentration," as generally used herein, is the lowest known concentration of a compound that is perceivable by the human sense of taste as sweet. The sweetness recognition threshold concentration is specific for a particular compound, and can vary based on temperature, matrix, ingredients and/or flavor system.

The term "sweetness enhancer" is synonymous with the terms "sweet taste potentiator," "sweetness potentiator," "sweetness amplifier," and "sweetness intensifier."

"Sweetener component", as used herein, refers to the compound or mixture of compounds that provides detectable sweetness when added to a consumable, i.e. the compound or mixture of sweet compounds present in the consumable in an amount above their sweetness recognition threshold concentration.

"Taste modulator", as used herein, refers to a compound that positively impacts the perception of a non-sucrose sweetener in a consumable (e.g. a beverage) in such a way that the consumable tastes more like a sucrose-sweetened beverage. For example, certain negative taste properties of non-sucrose sweeteners can be masked with taste modulators, e.g. bitterness, sourness, astringency, saltiness and metallic notes. In another example, mouthfeel can be improved. In still another example, sweetness linger can be decreased. In yet another example, sweetness onset can be increased. In a further example, sweetness onset can be improved. In a still further example, the bitterness linger can be improved.

### II. Enhancers/Taste Modulators

In one embodiment, dihydrochalcone compounds of the formulae described herein are sweetness enhancers. In another embodiment, dihydrochalcone compounds of the formulae described herein are taste modulators, with or without simultaneously acting as sweetness enhancers. That is, in some embodiments, a dihydrochalcone compound enhances sweetness and modulates one or more taste attributes of the sweetener. In other embodiments, a dihydrochalcone compound enhances the sweetness of the sweetener without modulating one or more taste attributes of the sweetener. In still other embodiments, a dihydrochalcone compound modulates one or more taste attributes of the sweetener without enhancing the sweetness of the sweetener.

Dihydrochalcone compounds of Formula I are useful as a sweetness enhancers and/or taste modulators: 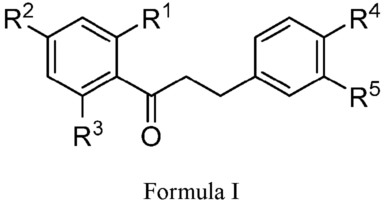 wherein R¹, R², R³, R⁴ and R⁵ are each independently selected from H, OH, OR⁶, alkyl and substituted alkyl, wherein R⁶ is selected from alkyl, substituted alkyl and a saccharide.

"Saccharide", as used herein, refers to monosaccharides, disaccharides, oligosaccharides and polysaccharides. A saccharide comprises at least one carbohydrate. Exemplary carbohydrates include, but are not limited to, sucrose, glyceraldehyde, dihydroxyacetone, erythrose, threose, erythrulose, arabinose, lyxose, ribose, xylose, ribulose, xylulose, allose, altrose, galactose, glucose, gulose, idose, mannose, talose, fructose, psicose, sorbose, tagatose, mannoheptulose, sedoheltulose, octolose, fucose, rhamnose, arabinose, turanose and sialose.

In a particular embodiment of the present invention, the dihydrochalcone is hesperetin dihydrochalcone:

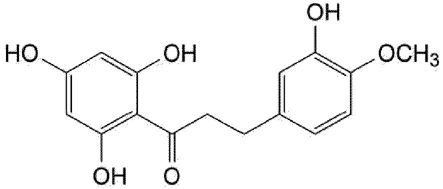

### 3-(3-Hydroxy-4-methoxyphenyl)-1-(2,4,6-trihydroxyphenyl)-1-propanone

In another particular embodiment of the present invention, the dihydrochalcone is hesperetin dihydrochalcone-4'-β-D-glucoside:

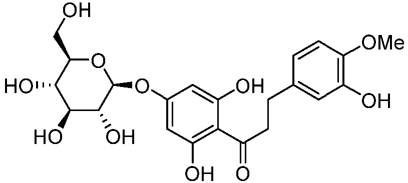

The dihydrochalcone compounds described herein can be provided in pure form or as part of mixture. The mixture can be an extract prepared from the plant or portion of a plant, or commercially available.

In one embodiment, the dihydrochalcone compound comprises at least about 50% by weight of a mixture, such as, for example, at least about 60%, at least about 70%, at least about 80%, at least about 90% or at least about 95%. In a more particular embodiment, the dihydrochalcone compound comprises at least about 96%, at least about 97%, at least about 98% or at least about 99% by weight of a mixture.

Mixtures of at least two dihydrochalcone compounds of the formulae described herein are also contemplated, such as, for example, at least three, at least four, at least five and at least six dihydrochalcone compounds. The at least two dihydrochalcone compounds can each independently be provided either in pure form or as part of a mixture. In one embodiment, the at least two dihydrochalcone compounds comprise at least about 50% by weight of a mixture, such as, for example, at least about 60%, at least about 70%, at least about 80%, at least about 90% or at least about 95%. In a more particular embodiment, the at least two dihydrochalcone compounds comprise at least about 96%, at least about 97%, at least about 98% or at least about 99% by weight of a mixture.

### III. Sweetener

The dihydrochalcone compounds of the present invention enhance and/or modulate the taste of at least one sweetener, particularly certain steviol glycoside and certain mogroside sweeteners.

### A. Steviol Glycoside Sweetener

In exemplary embodiments, the sweetener comprises at least one steviol glycoside, wherein the steviol glycoside is rebaudioside M, wherein the at least one steviol glycoside is present in a sweetening amount. "Sweetening amount", as used herein, refers to the amount of compound required to provide detectable sweetness when present in a consumable, e.g. a beverage.

The steviol glycoside can be natural, synthetic or a combination of natural and synthetic.

The steviol glycoside can be provided in pure form or as part of a mixture, i.e. a steviol glycoside blend.

Exemplary other steviol glycosides include, but are not limited to, rebaudioside D, rebaudioside A, rebaudioside N, rebaudioside O, rebaudioside E, steviolmonoside, steviolbioside, rubusoside, dulcoside B, dulcoside A, rebaudioside B, rebaudioside G, stevioside, rebaudioside C, rebaudioside F, rebaudioside I, rebaudioside H, rebaudioside L, rebaudioside K, rebaudioside J, rebaudioside M2, rebaudioside D2, rebaudioside S, rebaudioside T, rebaudioside U, rebaudioside V, rebaudioside W, rebaudioside Z1, rebaudioside Z2, rebaudioside IX, enzymatically glucosylated steviol glycosides and combinations thereof.

In certain embodiments, a steviol glycoside blend comprises at least about 5% of the steviol glycoside by weight, such as, for example, at least about 10%, at least about 20%, at least about 30%, at least about 40%, at least about 50%, at least about 60%, at least about 70%, at least about 80%, at least about 90%, at least about 95% or at least about 97%.

In exemplary embodiments, the steviol glycoside blend comprises at least about 50% of the steviol glycoside by weight, such as, for example, from about 50% to about 90%, from about 50% to about 80%, from about 50% to about 70%, from about 50% to about 60%, from about 60% to about 90%, from about 60% to about 80%, from about 60% to about 70%, from about 70% to about 90%, from about 70% to about 80% and from about 80% to about 90%.

In one embodiment, the sweetener is a steviol glycoside blend comprising rebaudioside M in a sweetening amount. In one embodiment, the steviol glycoside blend comprises at least about 5% rebaudioside M by weight, such as, for example, at least about 10%, at least about 20%, at least about 30%, at least about 40%, at least about 50%, at least about 60%, at least about 70%, at least about 80%, at least about 90%, at least about 95% or at least about 97%.

The steviol glycoside blend sweetener typically has a total steviol glycoside content of about 95% by weight or greater on a dry basis. The remaining 5% comprises other non-steviol glycoside compounds, e.g. by-products from extraction or purification processes. In some embodiments, the steviol glycoside blend sweetener has a total steviol glycoside content of about 96% or greater, about 97% or greater, about 98% or greater or about 99% or greater. "Total steviol glycoside content", as used herein, refers to the sum of the relative weight contributions of each steviol glycoside in a sample.

The weight ratio of the at least one sweetener described hereinabove to at least one dihydrochalcone of the formulae described herein can vary. Typically, the weight ratio of at least one sweetener to at least one dihydrochalcone is from about 500:1 to about 2:1, such as, for example, from about 100:1 to about 2:1, from about 50:1 to about 2:1, from about 25:1 to about 2:1, from about 10:1 to about 2:1, from about 5:1 to about 2:1, from about 500:1 to about 400:1, from about 500:1 to about 300:1, from about 500:1 to about 200: 1, from about 500:1 to about 100:1, from about 500:1 to about 50:1, from about 500:1 to about 25:1, from about 500:1 to about 10:1, from about 400:1 to about 300:1, from about 400:1 to about 200:1, from about 400:1 to about 100:1, from about 400:1 to about 50:1, from about 400:1 to about 25:1, from about 400:1 to about 10:1, from about 400:1 to about 6.67:1, from about 300:1 to about 200:1, from about 300:1 to about 100:1, from about 300:1 to about 50:1, from about 300:1 to about 25:1, from about 300:1 to about 10:1, from about 300:1 to about 6.67:2, from about 200:1 to about 100:1, from about 200:1 to about 50:1, from about 200:1 to about 25:1, from about 200:1 to about 10:1, from about 100:1 to about 50:1, from about 100:1 to about 25:1, from about 100:1 to about 10:1, from about 100:1 to about 6.67:1, from about 50:1 to about 25:1, from about 50:1 to about 25:1, from about 50:1 to about 10:1, from about 50:1 to about 6.65:1, from about 25:1 to about 10:1, from about 25:1 to about 6.67:1, from about 10:1 to about 6.67:1 and any range in between.

### B. Mogroside Sweetener

In one embodiment, the sweetener comprises at least one mogroside, wherein the mogroside is siamenoside I or the 1,6-α isomer of siamenoside I, and wherein the at least one mogroside is present in a sweetening amount. The mogroside can be natural, synthetic or a combination of natural and synthetic.

The mogroside can be provided in pure form or as part of mixture, i.e. a mogroside blend. Exemplary other mogrosides include, but are not limited to, any of grosmogroside I, mogroside IA, mogroside IE, 11-oxomogroside IA, mogroside II, mogroside II A, mogroside II B, mogroside II E, 7-oxomogroside II E, mogroside III, Mogroside IIIe, 11-oxomogroside IIIE, 11-deoxymogroside III, mogroside IV, Mogroside IVA 11-oxomogroside IV, 11-oxomogroside IVA, mogroside V, isomogroside V, 11-deoxymogroside V, 7-oxomogroside V, 11-oxomogroside V, isomogroside V, mogroside VI, mogrol, 11-oxomogrol, and combinations thereof.

Isomers of siamenoside I are disclosed in US20170119032, in particular the 1,6-α isomer of siamenoside I (Examples 7 and 10).

Additional exemplary mogrosides include those described in U.S. Patent Application Publication 2016039864. Other mogrosides include (3β,9β,10α,11α,24R)-3-[(4-O-β-D-glucospyranosyl-6-O-β-D-glucopyranosyl]-25-hydroxyl-9-methyl-19-norlanost-5-en-24-yl-[2-O-β-D-glucopyranosyl-6-O- β-D-glucopyranosyl]- β-D-glucopyranoside); (3β, 9β, 10α, 11α, 24R)-[(2-O- β-D-glucopyranosyl-6-O- β-D-glucopyranosyl- β-D- glucopyranosyl)oxy]-25-hydroxy-9-methyl-19-norlanost-5-en-24-yl-[2-O- β-D-glucopyranosyl-6-O-β-D-glucopyranosyl]-β-D-glucopyranoside); (3β, 9β, 10α, 11α, 24R)-[(2-O-β-D-glucopyranosyl-6-O-β-D-glucopyranosyl-β-D-glucopyranosyl)oxy]-25-hydroxy-9-methyl-19-norlanost-5-en-24-yl-[2-O-β-D-glucopyranosyl-6-O-β-D-glucopyranosyl]-β-D-glucopyranoside) and combinations thereof.

The mogroside blend comprises at least one mogroside in a sweetening amount.

In certain embodiments, a mogroside blend comprises at least about 5% of the mogroside by weight, such as, for example, at least about 10%, at least about 20%, at least about 30%, at least about 40%, at least about 50%, at least about 60%, at least about 70%, at least about 80%, at least about 90%, at least about 95% or at least about 97%.

In other embodiments, the mogroside blend has a total mogroside content of about 95% by weight or greater on a dry basis. In some embodiments, the mogroside blend sweetener has a total mogroside content of about 96% or greater, about 97% or greater, about 98% or greater or about 99% or greater. "Total mogroside content", as used herein, refers to the sum of the relative weight contributions of each mogroside in a sample.

In one embodiment, the sweetener is a mogroside blend comprising siamenoside I in a sweetening amount. In one embodiment, the mogroside blend comprises at least about 5% siamenoside I by weight, such as, for example, at least about 10%, at least about 20%, at least about 30%, at least about 40%, at least about 50%, at least about 60%, at least about 70%, at least about 80%, at least about 90%, at least about 95% or at least about 97%.

In another embodiment, the sweetener is a mogroside blend comprising the 1,6-α isomer of siamenoside I in a sweetening amount. In one embodiment, the mogroside blend comprises at least about 5% of the 1,6-α isomer of siamenoside I by weight, such as, for example, at least about 10%, at least about 20%, at least about 30%, at least about 40%, at least about 50%, at least about 60%, at least about 70%, at least about 80%, at least about 90%, at least about 95% or at least about 97%.

The weight ratio of the at least one sweetener described hereinabove to at least one dihydrochalcone of the formulae described herein can vary. Typically, the weight ratio of at least one sweetener to at least one dihydrochalcone is from about 500:1 to about 2:1, such as, for example, from about 100:1 to about 2:1, from about 50:1 to about 2:1, from about 25:1 to about 2:1, from about 10:1 to about 2:1, from about 5:1 to about 2:1, from about 500:1 to about 400:1, from about 500:1 to about 300:1, from about 500:1 to about 200: 1, from about 500:1 to about 100:1, from about 500:1 to about 50:1, from about 500:1 to about 25:1, from about 500:1 to about 10:1, from about 400:1 to about 300:1, from about 400:1 to about 200:1, from about 400:1 to about 100:1, from about 400:1 to about 50:1, from about 400:1 to about 25:1, from about 400:1 to about 10:1, from about 400:1 to about 6.67:1, from about 300:1 to about 200:1, from about 300:1 to about 100:1, from about 300:1 to about 50:1, from about 300:1 to about 25:1, from about 300:1 to about 10:1, from about 300:1 to about 6.67:2, from about 200:1 to about 100:1, from about 200:1 to about 50:1, from about 200:1 to about 25:1, from about 200:1 to about 10:1, from about 100:1 to about 50:1, from about 100:1 to about 25:1, from about 100:1 to about 10:1, from about 100:1 to about 6.67:1, from about 50:1 to about 25:1, from about 50:1 to about 25:1, from about 50:1 to about 10:1, from about 50:1 to about 6.65:1, from about 25:1 to about 10:1, from about 25:1 to about 6.67:1, from about 10:1 to about 6.67:1 and any range in between.

### III. Sweetener Component

In one embodiment, one of the sweeteners described above is the only sweetener in the beverage. In other embodiments, a beverage comprises a sweetener component containing one of the sweeteners described above and one or more additional sweeteners.

Typically, a sweetener of the present invention comprises at least about 50% by weight of the sweetener component, such as for example, at least about 60%, at least about 70%, at least about 80%, at least about 90% and at least about 95%. In a more particular embodiment, a sweetener of the present invention comprises at least about 96%, at least about 97%, at least about 98% or at least about 99% of the sweetener component.

The additional sweetener used in the sweetener component can be any known sweetener, e.g. a natural sweetener, a natural high potency sweetener, a synthetic sweetener.

As used herein, the phrase "natural high potency sweetener" refers to any sweetener found naturally in nature and characteristically has a sweetness potency greater than sucrose, fructose, or glucose, yet has less calories. The natural high potency sweetener can be provided as a pure compound or, alternatively, as part of an extract. As used herein, the phrase "synthetic sweetener" refers to any composition which is not found naturally in nature and characteristically has a sweetness potency greater than sucrose, fructose, or glucose, yet has less calories.

In other embodiments, the additional sweetener is at least one carbohydrate sweetener. Suitable carbohydrate sweeteners are selected from, but not limited to, the group consisting of sucrose, glyceraldehyde, dihydroxyacetone, erythrose, threose, erythrulose, arabinose, lyxose, ribose, xylose, ribulose, xylulose, allose, altrose, galactose, glucose, gulose, idose, mannose, talose, fructose, psicose, sorbose, tagatose, mannoheptulose, sedoheltulose, octolose, fucose, rhamnose, arabinose, turanose, sialose and combinations thereof.

In other embodiments, the sweetener component does not comprise a carbohydrate sweetener.

Other suitable additional sweeteners include siamenoside I, monatin and its salts (monatin SS, RR, RS, SR), curculin, glycyrrhizic acid and its salts, thaumatin, monellin, mabinlin, brazzein, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, steviolbioside and cyclocarioside I, sugar alcohols such as erythritol, sucralose, potassium acesulfame, acesulfame acid and salts thereof, aspartame, alitame, saccharin and salts thereof, neohesperidin dihydrochalcone, cyclamate, cyclamic acid and salts thereof, neotame, advantame, glucosylated steviol glycosides (GSGs) and combinations thereof.

In one embodiment, the additional sweetener is a caloric sweetener or mixture of caloric sweeteners. In another embodiment, the caloric sweetener is selected from sucrose, fructose, glucose, high fructose corn/starch syrup, a beet sugar, a cane sugar, and combinations thereof.

In another embodiment, the additional sweetener is a rare sugar selected from sorbose, lyxose, ribulose, xylose, xylulose, D-allose, L-ribose, D-tagatose, L-glucose, L-fucose, L-arabinose, turanose and combinations thereof. The rare sugars can be present in the sweetener compositions in an amount from about 0.5% to about 3.0%, such as, for example, about 0.5% to about 2.5%, about 0.5% to about 2.0%, about 0.5% to about 1.5%, about 0.5% to about 1.0%, about 1.0% to about 3.0%, about 1.0% to about 2.5%, about 1.0% to about 2.0%, about 1.0% to about 1.5%, about 2.0% to about 3.0% and about 2.0% to about 2.5%.

### IV. Compositions

Described herein are compositions comprising at least one sweetener described herein and at least one dihydrochalcone of the formulae described herein. Also described is a composition comprising a sweetener component comprising at least one sweetener described herein and at least one dihydrochalcone of the formulae described herein.

The at least one dihydrochalcone described herein may be present in a composition in an amount such that, when the composition is added to a consumable, the SE of the consumable is enhanced by at least about 1.2-fold compared to the sucrose equivalence of the consumable in the absence of the at least one dihydrochalcone, such as, for example, at least about 1.5-fold, at least about 1.7-fold, at least about 2.0-fold, at least about 2.5-fold, at least about 3.0-fold, or at least about 4.0-fold.

The amount of sucrose, and thus another measure of sweetness, in a reference solution may be described in degrees Brix (°Bx). One degree Brix is 1 gram of sucrose in 100 grams of solution and represents the strength of the solution as percentage by weight (% w/w) (strictly speaking, by mass).

The at least one dihydrochalcone described herein may be present in a composition in an amount such that, when the composition is added to a consumable, the consumable has a sweetness equivalent to about 8 degrees Brix, such as, for example, about 8 degrees Brix, about 9 degrees Brix, about 10 degrees Brix, about 11 degrees Brix or about 12 degrees Brix.

The at least one dihydrochalcone described herein may be present in a composition in an amount such that, when the composition added to a consumable, the Brix of the consumable increases by at least 1 degree, such as, for example, at least 2 degrees Brix, at least 3 degrees Brix, at least 4 degrees Brix or at least 5 degrees Brix.

The at least one dihydrochalcone of the formulae described herein may be present in a composition in an amount effective such that, when the composition is added to a consumable, one or more taste attributes of the sweetener is modulated making the consumable taste more like a sucrose-sweetened consumable compared to the same one or more taste attributes of the consumable in the absence of the at least one dihydrochalcone. Exemplary taste attribute modulations include decreasing or eliminating bitterness, decreasing or eliminating bitter linger, decreasing or eliminating sourness, decreasing or eliminating astringency, decreasing or eliminating saltiness, decreasing or eliminating metallic notes, improving mouthfeel, decreasing or eliminating sweetness linger, and increasing sweetness onset. Multiple taste attributes of the sweetener can be modulated simultaneously, such that the consumable, overall, has more sucrose-sweetened characteristics. Methods of quantifying improvement in sucrose-sweetened characteristics are known in the art and includes, e.g., taste testing and histogram mapping.

It should be noted that the comparisons are between (i) the consumable containing the at least one sweetener and at least one dihydrochalcone and (ii) the consumable containing the at least one sweetener - but not the at least one dihydrochalcone.

### V. Consumables

The present invention provides a consumable comprising at least one sweetener described hereinabove and at least one dihydrochalcone described herein, wherein the consumable is a beverage. The present invention also provides a consumable comprising a sweetener component comprising at least one sweetener described herein and at least one dihydrochalcone described herein, wherein the consumable is a beverage.

The at least one dihydrochalcone compound of the formulae described is typically present in the consumable in an amount effective to enhance the sweetness thereof and/or modulate one or more taste attributes of the sweetener to make the consumable taste more like a sucrose-sweetened consumable. In one embodiment, the at least one dihydrochalcone is present in an amount at or below the dihydrochalcone's sweetness recognition threshold concentration.

The at least one dihydrochalcone compound of the formulae described is present in the consumable in an amount effective to enhance the sweetness of the consumable. Typically, the at least one dihydrochalcone is present in the consumable in an amount effective to enhance the sucrose equivalence (SE) of the consumable by at least 1.2-fold when compared to the SE of the consumable in the absence of the at least one dihydrochalcone, such as for example, at least about 1.3-fold, at least about 1.4-fold, at least about 1.5-fold, at least about 1.6-fold, at least about 1.7-fold, at least about 1.8-fold, at least about 1.9-fold and at least about 2.0-fold.

In some embodiments, the at least one dihydrochalcone described herein is present in the consumable in an amount effective to provide a sweetness equivalent to about 8 degrees Brix, such as, for example, about 8 degrees Brix, about 9 degrees Brix, about 10 degrees Brix, about 11 degrees Brix or about 12 degrees Brix.

In other embodiments, the at least one dihydrochalcone described herein is present in the consumable in an amount effective to increase the degrees Brix of the consumable by at least 1 degree compared to the degrees Brix of the consumable in the absence of the at least one dihydrochalcone, such as, for example, at least 2 degrees Brix, at least 3 degrees Brix, at least 4 degrees Brix or at least 5 degrees Brix.

In other embodiments, the at least one dihydrochalcone of the formulae described is present in the consumable in an amount effective to modulate one or more taste attributes of the sweetener to make the consumable taste more like a sucrose-sweetened consumable. Exemplary taste attribute modulations include decreasing or eliminating bitterness, decreasing or eliminating bitter linger, decreasing or eliminating sourness, decreasing or eliminating astringency, decreasing or eliminating saltiness, decreasing or eliminating metallic notes, improving mouthfeel, decreasing or eliminating sweetness linger, and increasing sweetness onset. Multiple taste attributes of the sweetener can be modulated simultaneously, such that the consumable, overall, has more sucrose-sweetened characteristics. Methods of quantifying improvement in sucrose-sweetened characteristics are known in the art and includes taste testing and histogram mapping.

The particular concentration of the at least one dihydrochalcone compound of the formulae described herein and the sweetener will vary depending on the particular dihydrochalcone(s) and sweetener(s).

In one embodiment, the at least one dihydrochalcone described herein is present in the consumable in a concentration from about 1 ppm to about 50 ppm, such as, for example, from about 1 ppm to about 45 ppm, from about 1 ppm to about 40 ppm, from about 1 ppm to about 35 ppm, from about 1 ppm to about 30 ppm, from about 1 ppm to about 25 ppm, from about 1 ppm to about 20 ppm, from about 1 ppm to about 15 ppm, from about 1 ppm to about 10 ppm and from about 1 ppm to about 5 ppm. In another embodiment, the at least one dihydrochalcone is present in the consumable in a concentration from about 5 ppm to about 40 ppm, such as, for example, from about 5 ppm to about 35 ppm, from about 5 ppm to about 20 ppm, from about 20 to about 40 ppm, from about 20 ppm to about 30 ppm or from about 30 ppm to about 40 ppm.

In one embodiment, the at least one sweetener described herein is present in the consumable in a concentration from about 50 ppm to about 600 ppm, such as, for example, about 50 ppm to about 500 ppm, from about 50 ppm to about 400 ppm, from about 50 ppm to about 300 ppm, from about 50 ppm to about 300 ppm, from about 50 ppm to about 200 ppm, from about 50 ppm to about 100 ppm, about 100 ppm to about 600 ppm, about 100 ppm to about 500 ppm, about 100 ppm to about 400 ppm, about 100 ppm to about 300 ppm, about 100 ppm to about 200 ppm, about 200 ppm to about 600 ppm, about 200 ppm to about 500 ppm, about 200 ppm to about 400 ppm, about 200 ppm to about 300 ppm, about 300 ppm to about 600 ppm, about 300 ppm to about 500 ppm, about 300 ppm to about 400 ppm, about 400 ppm to about 600 ppm, about 400 ppm to about 500 ppm and about 500 ppm to about 600 ppm.

The weight ratio of at least one sweetener described herein to the at least one dihydrochalcone of the formulae described herein can also vary, as discussed above.

In a particular embodiment, a consumable comprises at least one dihydrochalcone of the formulae described herein and at least one sweetener described herein, wherein the at least one dihydrochalcone is present in an amount effective to enhance the SE of the consumable by at least about 1.2-fold when compared to the SE of the consumable in the absence of the at least one dihydrochalcone.

In one embodiment, the consumable with enhanced sweetness has a SE of about 2% (w/v) or greater, such as, for example, about 3% or greater, about 4% or greater, about 5% or greater, about 6% or greater, about 7% or greater, about 8% or greater, about 9% or greater, about 10% or greater, about 11% or greater, about 12% or greater, about 13% or greater or about 14% or greater.

In another embodiment, the consumable with enhanced sweetness has a Brix level of about 3 to about 12, such as, for example, about 3 degrees Brix or greater, about 4 degrees Brix or greater, about 5 degrees Brix or greater, about 5 degrees Brix or greater, about 7 degrees Brix or greater, about 8 degrees Brix or greater, about 9 degrees Brix or greater, about 10 degrees Brix or greater and about 11 degrees Brix or greater.

In the present invention the consumable is a beverage. Other exemplary consumables include, but are not limited to edible gel mixes and compositions, dental compositions, foodstuffs (confections, condiments, chewing gum, cereal compositions, baked goods, dairy products, and tabletop sweetener compositions).

### Edible Gel Mixes and Edible Gel Compositions

Edible gels are gels that can be eaten. A gel is a colloidal system in which a network of particles spans the volume of a liquid medium. Although gels mainly are composed of liquids, and thus exhibit densities similar to liquids, gels have the structural coherence of solids due to the network of particles that spans the liquid medium. For this reason, gels generally appear to be solid, jelly-like materials. Gels can be used in a number of applications. For example, gels can be used in foods, paints, and adhesives.

Non-limiting examples of edible gel compositions for use in particular embodiments include gel desserts, puddings, jellies, pastes, trifles, aspics, marshmallows, gummy candies, or the like. Edible gel mixes generally are powdered or granular solids to which a fluid may be added to form an edible gel composition. Non-limiting examples of fluids for use in particular embodiments include water, dairy fluids, dairy analogue fluids, juices, alcohol, alcoholic beverages, and combinations thereof. Non-limiting examples of dairy fluids which may be used in particular embodiments include milk, cultured milk, cream, fluid whey, and mixtures thereof. Non-limiting examples of dairy analogue fluids which may be used in particular embodiments include, for example, soy milk and non-dairy coffee whitener. Because edible gel products found in the marketplace typically are sweetened with sucrose, it is desirable to sweeten edible gels with an alternative sweetener in order provide a low-calorie or non-calorie alternative.

### Dental Compositions

Dental compositions generally comprise an active dental substance and a base material. The dental composition may be in the form of any oral composition used in the oral cavity such as mouth freshening agents, gargling agents, mouth rinsing agents, toothpaste, tooth polish, dentifrices, mouth sprays, teeth-whitening agent, dental floss, and the like, for example.

### Confections

As referred to herein, "confection" can mean a sweet, a lollie, a confectionery, or similar term. The confection generally contains a base composition component and a sweetener component. The confection may be in the form of any food that is typically perceived to be rich in sugar or is typically sweet. According to particular embodiments of the present invention, the confections may be bakery products such as pastries; desserts such as yogurt, jellies, drinkable jellies, puddings, Bavarian cream, blancmange, cakes, brownies, mousse and the like, sweetened food products eaten at tea time or following meals; frozen foods; cold confections, e.g. types of ice cream such as ice cream, ice milk, lacto-ice and the like (food products in which sweeteners and various other types of raw materials are added to milk products, and the resulting mixture is agitated and frozen), and ice confections such as sherbets, dessert ices and the like (food products in which various other types of raw materials are added to a sugary liquid, and the resulting mixture is agitated and frozen); general confections, e.g., baked confections or steamed confections such as crackers, biscuits, buns with bean-jam filling, halvah, alfajor, and the like; rice cakes and snacks; table top products; general sugar confections such as chewing gum (e.g. including compositions which comprise a substantially water-insoluble, chewable gum base, such as chicle or substitutes thereof, including jetulong, guttakay rubber or certain comestible natural synthetic resins or waxes), hard candy, soft candy, mints, nougat candy, jelly beans, fudge, toffee, taffy, Swiss milk tablet, licorice candy, chocolates, gelatin candies, marshmallow, marzipan, divinity, cotton candy, and the like; sauces including fruit flavored sauces, chocolate sauces and the like; edible gels; crèmes including butter crèmes, flour pastes, whipped cream and the like; jams including strawberry jam, marmalade and the like; and breads including sweet breads and the like or other starch products, and combinations thereof.

### Condiment Compositions

Condiments, as used herein, are compositions used to enhance or improve the flavor of a food or beverage. Non-limiting examples of condiments include ketchup (catsup); mustard; barbecue sauce; butter; chili sauce; chutney; cocktail sauce; curry; dips; fish sauce; horseradish; hot sauce; jellies, jams, marmalades, or preserves; mayonnaise; peanut butter; relish; remoulade; salad dressings (e.g., oil and vinegar, Caesar, French, ranch, bleu cheese, Russian, Thousand Island, Italian, and balsamic vinaigrette), salsa; sauerkraut; soy sauce; steak sauce; syrups; tartar sauce; and Worcestershire sauce.

The condiment composition optionally may include other natural and/or synthetic high-potency sweeteners, bulk sweeteners, pH modifying agents (e.g., lactic acid, citric acid, phosphoric acid, hydrochloric acid, acetic acid, and combinations thereof), fillers, functional agents (e.g., pharmaceutical agents, nutrients, or components of a food or plant), flavorings, colorings, or combinations thereof.

### Chewing Gum Compositions

Chewing gum compositions generally comprise a water-soluble portion and a water-insoluble chewable gum base portion. The water soluble portion dissipates with a portion of the flavoring agent over a period of time during chewing while the insoluble gum base portion is retained in the mouth. The insoluble gum base generally determines whether a gum is considered chewing gum, bubble gum, or a functional gum.

### Cereal Compositions

Cereal compositions typically are eaten either as staple foods or as snacks. Non-limiting examples of cereal compositions for use in particular embodiments include ready-to-eat cereals as well as hot cereals. Ready-to-eat cereals are cereals which may be eaten without further processing (i.e. cooking) by the consumer. Examples of ready-to-eat cereals include breakfast cereals and snack bars. Breakfast cereals typically are processed to produce a shredded, flaky, puffy, or extruded form. Breakfast cereals generally are eaten cold and are often mixed with milk and/or fruit. Snack bars include, for example, energy bars, rice cakes, granola bars, and nutritional bars. Hot cereals generally are cooked, usually in either milk or water, before being eaten. Non-limiting examples of hot cereals include grits, porridge, polenta, rice, and rolled oats.

Cereal compositions generally comprise at least one cereal ingredient. As used herein, the term "cereal ingredient" denotes materials such as whole or part grains, whole or part seeds, and whole or part grass. Non-limiting examples of cereal ingredients for use in particular embodiments include maize, wheat, rice, barley, bran, bran endosperm, bulgur, soghums, millets, oats, rye, triticale, buchwheat, fonio, quinoa, bean, soybean, amaranth, teff, spelt, and kaniwa.

### Baked Goods

"Baked goods," as used herein, include ready to eat and all ready to bake products, flours, and mixes requiring preparation before serving. Non-limiting examples of baked goods include cakes, crackers, cookies, brownies, muffins, rolls, bagels, donuts, strudels, pastries, croissants, biscuits, bread, bread products, and buns.

Baked goods in accordance with particular embodiments generally comprise a combination of sweetener, water, and fat.

### Dairy Products

Dairy products and processes for making dairy products suitable for use in this invention are well known to those of ordinary skill in the art. Dairy products, as used herein, comprise milk or foodstuffs produced from milk. Non-limiting examples of dairy products suitable for use in embodiments of this invention include milk, milk cream, sour cream, crème fraiche, buttermilk, cultured buttermilk, milk powder, condensed milk, evaporated milk, butter, cheese, cottage cheese, cream cheese, yogurt, ice cream, frozen custard, frozen yogurt, gelato, vla, piima, filmjölk, kajmak, kephir, viili, kumiss, airag, ice milk, casein, ayran, lassi, khoa, or combinations thereof.

In particular embodiments of this invention, the processing of the dairy product from raw milk generally comprises the steps of pasteurizing, creaming, and homogenizing. Although raw milk may be consumed without pasteurization, it usually is pasteurized to destroy harmful microorganisms such as bacteria, viruses, protozoa, molds, and yeasts. Pasteurizing generally comprises heating the milk to a high temperature for a short period of time to substantially reduce the number of microorganisms, thereby reducing the risk of disease.

Particular embodiments of this invention comprise dairy products produced from milk by additional processing steps. As described above, cream may be skimmed from the top of milk or separated from the milk using machine-centrifuges. In a particular embodiment, the dairy product comprises sour cream, a dairy product rich in fats that is obtained by fermenting cream using a bacterial culture. The bacteria produce lactic acid during fermentation, which sours and thickens the cream. In another particular embodiment, the dairy product comprises crème fraiche, a heavy cream slightly soured with bacterial culture in a similar manner to sour cream. Crème fraiche ordinarily is not as thick or as sour as sour cream. In yet another particular embodiment, the dairy product comprises cultured buttermilk. Cultured buttermilk is obtained by adding bacteria to milk. The resulting fermentation, in which the bacterial culture turns lactose into lactic acid, gives cultured buttermilk a sour taste. Although it is produced in a different manner, cultured buttermilk generally is similar to traditional buttermilk, which is a by-product of butter manufacture.

According to other particular embodiments of this invention, the dairy products comprise milk powder, condensed milk, evaporated milk, or combinations thereof. Milk powder, condensed milk, and evaporated milk generally are produced by removing water from milk. In a particular embodiment, the dairy product comprises a milk powder comprising dried milk solids with a low moisture content. In another particular embodiment, the dairy product comprises condensed milk. Condensed milk generally comprises milk with a reduced water content and added sweetener, yielding a thick, sweet product with a long shelf-life. In yet another particular embodiment, the dairy product comprises evaporated milk. Evaporated milk generally comprises fresh, homogenized milk from which about 60 % of the water has been removed, that has been chilled, fortified with additives such as vitamins and stabilizers, packaged, and finally sterilized. According to another particular embodiment of this invention, the dairy product comprises a dry creamer and a steviol glycoside blend of the present invention or a sweetener composition comprising the same.

In another particular embodiment, the dairy product provided herein comprises butter. Butter generally is made by churning fresh or fermented cream or milk. Butter generally comprises butterfat surrounding small droplets comprising mostly water and milk proteins. The churning process damages the membranes surrounding the microscopic globules of butterfat, allowing the milk fats to conjoin and to separate from the other parts of the cream. In yet another particular embodiment, the dairy product comprises buttermilk, which is the sour-tasting liquid remaining after producing butter from full-cream milk by the churning process.

In still another particular embodiment, the dairy product comprises cheese, a solid foodstuff produced by curdling milk using a combination of rennet or rennet substitutes and acidification. Rennet, a natural complex of enzymes produced in mammalian stomachs to digest milk, is used in cheese-making to curdle the milk, causing it to separate into solids known as curds and liquids known as whey. Generally, rennet is obtained from the stomachs of young ruminants, such as calves; however, alternative sources of rennet include some plants, microbial organisms, and genetically modified bacteria, fungus, or yeast. In addition, milk may be coagulated by adding acid, such as citric acid. Generally, a combination of rennet and/or acidification is used to curdle the milk. After separating the milk into curds and whey, some cheeses are made by simply draining, salting, and packaging the curds. For most cheeses, however, more processing is needed. Many different methods may be used to produce the hundreds of available varieties of cheese. Processing methods include heating the cheese, cutting it into small cubes to drain, salting, stretching, cheddaring, washing, molding, aging, and ripening. Some cheeses, such as the blue cheeses, have additional bacteria or molds introduced to them before or during aging, imparting flavor and aroma to the finished product. Cottage cheese is a cheese curd product with a mild flavor that is drained but not pressed so that some whey remains. The curd is usually washed to remove acidity. Cream cheese is a soft, mild-tasting, white cheese with a high fat content that is produced by adding cream to milk and then curdling to form a rich curd. Alternatively, cream cheese can be made from skim milk with cream added to the curd. It should be understood that cheese, as used herein, comprises all solid foodstuff produced by the curdling milk.

In another particular embodiment of this invention, the dairy product comprises yogurt. Yogurt generally is produced by the bacterial fermentation of milk. The fermentation of lactose produces lactic acid, which acts on proteins in milk to give the yogurt a gel-like texture and tartness. In particularly desirable embodiments, the yogurt may be sweetened with a sweetener and/or flavored. Non-limiting examples of flavorings include, but are not limited to, fruits (e.g., peach, strawberry, banana), vanilla, and chocolate. Yogurt, as used herein, also includes yogurt varieties with different consistencies and viscosities, such as dahi, dadih or dadiah, labneh or labaneh, bulgarian, kefir, and matsoni. In another particular embodiment, the dairy product comprises a yogurt-based beverage, also known as drinkable yogurt or a yogurt smoothie. In particularly desirable embodiments, the yogurt-based beverage may comprise sweeteners, flavorings, other ingredients, or combinations thereof.

Other dairy products beyond those described herein may be used in particular embodiments of this invention. Such dairy products are well known to those of ordinary skill in the art, non-limiting examples of which include milk, milk and juice, coffee, tea, vla, piima, filmjolk, kajmak, kephir, viili, kumiss, airag, ice milk, casein, ayran, lassi, and khoa.

According to particular embodiments of this invention, the dairy compositions also may comprise other additives. Non-limiting examples of suitable additives include sweeteners and flavorants such as chocolate, strawberry, and banana. Particular embodiments of the dairy compositions provided herein also may comprise additional nutritional supplements such as vitamins (e.g., vitamin D) and minerals (e.g., calcium) to improve the nutritional composition of the milk.

### Tabletop Sweetener Compositions

The tabletop sweetener can further include at least one bulking agent, additive, anti-caking agent, functional ingredient or combination thereof.

Suitable "bulking agents" include, but are not limited to, maltodextrin (10 DE, 18 DE, or 5 DE), corn syrup solids (20 or 36 DE), sucrose, fructose, glucose, invert sugar, sorbitol, xylose, ribulose, mannose, xylitol, mannitol, galactitol, erythritol, maltitol, lactitol, isomalt, maltose, tagatose, lactose, inulin, glycerol, propylene glycol, polyols, polydextrose, fructooligosaccharides, cellulose and cellulose derivatives, and the like, and mixtures thereof. Additionally, in accordance with still other embodiments of the invention, granulated sugar (sucrose) or other caloric sweeteners such as crystalline fructose, other carbohydrates, or sugar alcohol can be used as a bulking agent due to their provision of good content uniformity without the addition of significant calories.

As used herein, the phrase "anti-caking agent" and "flow agent" refer to any composition which assists in content uniformity and uniform dissolution. In accordance with particular embodiments, non-limiting examples of anti-caking agents include cream of tartar, calcium silicate, silicon dioxide, microcrystalline cellulose (Avicel, FMC BioPolymer, Philadelphia, Pennsylvania), and tricalcium phosphate. In one embodiment, the anti-caking agents are present in the tabletop sweetener composition in an amount from about 0.001 to about 3 % by weight of the tabletop sweetener composition.

The tabletop sweetener compositions can be packaged in any form known in the art. Non-limiting forms include, but are not limited to, powder form, granular form, packets, tablets, sachets, pellets, cubes, solids, and liquids.

In one embodiment, the tabletop sweetener composition is a single-serving (portion control) packet comprising a dry-blend. Dry-blend formulations generally may comprise powder or granules. Although the tabletop sweetener composition may be in a packet of any size, an illustrative non-limiting example of conventional portion control tabletop sweetener packets are approximately 2.5 by 1.5 inches and hold approximately 1 gram of a sweetener composition having a sweetness equivalent to 2 teaspoons of granulated sugar (~ 8 g). In a particular embodiment, a dry-blend tabletop sweetener formulation may contain a sweetener an amount from about 1 % (w/w) to about 10 % (w/w).

Solid tabletop sweetener embodiments include cubes and tablets. A non-limiting example of conventional cubes are equivalent in size to a standard cube of granulated sugar, which is approximately 2.2 x 2.2 x 2.2 cm³ and weigh approximately 8 g. In one embodiment, a solid tabletop sweetener is in the form of a tablet or any other form known to those skilled in the art.

A tabletop sweetener composition also may be embodied in the form of a liquid, wherein a steviol glycoside blend of the present invention or a sweetener composition comprising the same is combined with a liquid carrier. Suitable non-limiting examples of carrier agents for liquid tabletop sweeteners include water, alcohol, polyol, glycerin base or citric acid base dissolved in water, and mixtures thereof. The sweetness equivalent of a tabletop sweetener composition for any of the forms described herein or known in the art may be varied to obtain a desired sweetness profile. For example, a tabletop sweetener composition may comprise a sweetness comparable to that of an equivalent amount of standard sugar. In another embodiment, the tabletop sweetener composition may comprise a sweetness of up to 100 times that of an equivalent amount of sugar. In another embodiment, the tabletop sweetener composition may comprise a sweetness of up to 90 times, 80 times, 70 times, 60 times, 50 times, 40 times, 30 times, 20 times, 10 times, 9 times, 8 times, 7 times, 6 times, 5 times, 4 times, 3 times, and 2 times that of an equivalent amount of sugar.

### Beverage and Beverage Products

In the present invention, the consumable is a beverage or beverage product.

"Beverage product", as used herein, is a ready-to-drink beverage, a beverage concentrate, a beverage syrup, or a powdered beverage. Suitable ready-to-drink beverages include carbonated and non-carbonated beverages. Carbonated beverages include, but are not limited to, frozen carbonated beverages, enhanced sparkling beverages, cola, fruit-flavored sparkling beverages (e.g. lemon-lime, orange, grape, strawberry and pineapple), ginger-ale, soft drinks and root beer. Non-carbonated beverages include, but are not limited to, fruit juice, fruit-flavored juice, juice drinks, nectars, vegetable juice, vegetable-flavored juice, sports drinks, energy drinks, enhanced water drinks, enhanced water with vitamins, near water drinks (e.g., water with natural or synthetic flavorants), coconut water, tea type drinks (e.g. black tea, green tea, red tea, oolong tea), coffee, cocoa drink, beverage containing milk components (e.g. milk beverages, coffee containing milk components, café au lait, milk tea, fruit milk beverages), beverages containing cereal extracts and smoothies.

Beverage concentrates and beverage syrups are prepared with an initial volume of liquid matrix (e.g. water) and the desired beverage ingredients. Full strength beverages are then prepared by adding further volumes of water. Powdered beverages are prepared by dry-mixing all of the beverage ingredients in the absence of a liquid matrix. Full strength beverages are then prepared by adding the full volume of water.

Beverages comprise a matrix, i.e. the basic ingredient in which the ingredients - including the compositions of the present invention - are dissolved. In one embodiment, a beverage comprises water of beverage quality as the matrix, such as, for example deionized water, distilled water, reverse osmosis water, carbon-treated water, purified water, demineralized water and combinations thereof, can be used. Additional suitable matrices include, but are not limited to phosphoric acid, phosphate buffer, citric acid, citrate buffer and carbon-treated water.

The beverage or beverage product can further include at least one additional sweetener. Any of the sweeteners detailed herein can be used, including natural, non-natural, or synthetic sweeteners. In certain embodiments, the beverage or beverage product does not contain a carbohydrate sweetener.

In one embodiment, the beverage or beverage products comprise a rare sugar - either as part of the sweetener component or added to the beverage separately. Suitable rare sugars include, but are not limited to, allulose, sorbose, lyxose, ribulose, xylose, xylulose, D-allose, L-ribose, D-tagatose, L-glucose, L-fucose, L-arabinose, turanose and combinations thereof. The rare sugars can be present in beverage in an amount from about 0.5% to about 3.0%, such as, for example, about 0.5% to about 2.5%, about 0.5% to about 2.0%, about 0.5% to about 1.5%, about 0.5% to about 1.0%, about 1.0% to about 3.0%, about 1.0% to about 2.5%, about 1.0% to about 2.0%, about 1.0% to about 1.5%, about 2.0% to about 3.0% and about 2.0% to about 2.5%. In a particular embodiment, the rare sugar is allulose.

The beverage or beverage product can contain additives including, but not limited to, carbohydrates, polyols, amino acids and their corresponding salts, poly-amino acids and their corresponding salts, sugar acids and their corresponding salts, nucleotides, organic acids, inorganic acids, organic salts including organic acid salts and organic base salts, inorganic salts, bitter compounds, caffeine, flavorants and flavoring ingredients, astringent compounds, proteins or protein hydrolysates, surfactants, emulsifiers, weighing agents, juice, dairy, cereal and other plant extracts, flavonoids, alcohols, polymers and combinations thereof. Any suitable additive described herein can be used.

The beverage or beverage product can contain one or more functional ingredients, detailed herein. Functional ingredients include, but are not limited to, vitamins, minerals, antioxidants, preservatives, glucosamine, polyphenols and combinations thereof. Any suitable functional ingredient described herein can be used.

It is contemplated that the pH of the consumable, such as, for example, a beverage, does not materially or adversely affect the taste of the sweetener. A non-limiting example of the pH range of the beverage may be from about 1.8 to about 10. A further example includes a pH range from about 2 to about 5. In a particular embodiment, the pH of beverage can be from about 2.5 to about 4.2. On of skill in the art will understand that the pH of the beverage can vary based on the type of beverage. Dairy beverages, for example, can have pHs greater than 4.2.

The titratable acidity of a beverage may, for example, range from about 0.01 to about 1.0% by weight of beverage.

In one embodiment, the sparkling beverage product has an acidity from about 0.01 to about 1.0% by weight of the beverage, such as, for example, from about 0.05% to about 0.25% by weight of beverage.

The carbonation of a sparkling beverage product has 0 to about 2% (w/w) of carbon dioxide or its equivalent, for example, from about 0.1 to about 1.0% (w/w).

The beverage can be caffeinated or non-caffeinated.

The temperature of a beverage may, for example, range from about 4°C to about 100 °C, such as, for example, from about 4°C to about 25°C.

The beverage can be a full-calorie beverage that has up to about 120 calories per 8 oz serving.

The beverage can be a mid-calorie beverage that has up to about 60 calories per 8 oz. serving.

The beverage can be a low-calorie beverage that has up to about 40 calories per 8 oz. serving.

The beverage can be a zero-calorie that has less than about 5 calories per 8 oz. serving.

In a particular embodiment, the consumable is a cola beverage. The cola beverage can be a low-, mid- or zero-calorie beverage.

In some embodiments, the cola beverage further comprises allulose and/or erythritol.

The concentration of the at least one dihydrochalcone described herein and the sweetener can vary based on the identity of each.

In one embodiment, the at least one dihydrochalcone described herein is present in the beverage in a concentration from about 1 ppm to about 50 ppm, such as, for example, from about 1 ppm to about 45 ppm, from about 1 ppm to about 40 ppm, from about 1 ppm to about 35 ppm, from about 1 ppm to about 30 ppm, from about 1 ppm to about 25 ppm, from about 1 ppm to about 20 ppm, from about 1 ppm to about 15 ppm, from about 1 ppm to about 10 ppm and from about 1 ppm to about 5 ppm. In another embodiment, the at least one dihydrochalcone is present in the beverage in a concentration from about 5 ppm to about 50 ppm, from about 5 ppm to about 45 ppm, from about 5 ppm to about 40 ppm, from about 5 ppm to about 35 ppm, from about 5 ppm to about 30 ppm, from about 10 ppm to about 50 ppm, from about 10 ppm to about 40 ppm, from about 10 ppm to about 30 ppm, from about 15 ppm to about 50 ppm, from about 15 ppm to about 45 ppm, from about 15 ppm to about 40 ppm, from about 14 ppm to about 35 ppm, from about 15 ppm to about 30 ppm, from about 20 ppm to about 50 ppm, from about 20 ppm to about 40 ppm, from about 20 ppm to about 30 ppm and from about 25 ppm to about 30 ppm.

In one embodiment, the at least one sweetener described herein is present in the beverage in a concentration from about 50 ppm to about 600 ppm, such as, for example, about 50 ppm to about 500 ppm, from about 50 ppm to about 400 ppm, from about 50 ppm to about 300 ppm, from about 50 ppm to about 300 ppm, from about 50 ppm to about 200 ppm, from about 50 ppm to about 100 ppm, about 100 ppm to about 600 ppm, about 100 ppm to about 500 ppm, about 100 ppm to about 400 ppm, about 100 ppm to about 300 ppm, about 100 ppm to about 200 ppm, about 200 ppm to about 600 ppm, about 200 ppm to about 500 ppm, about 200 ppm to about 400 ppm, about 200 ppm to about 300 ppm, about 300 ppm to about 600 ppm, about 300 ppm to about 500 ppm, about 300 ppm to about 400 ppm, about 400 ppm to about 600 ppm, about 400 ppm to about 500 ppm and about 500 ppm to about 600 ppm.

In a particular embodiment, the present invention provides a beverage comprising hesperetin dihydrochalcone and a sweetener comprising a sweetening amount of rebaudioside M. The concentration of the hesperetin dihydrochalcone is from about 1 ppm to about 50 ppm, more particularly from about 5 ppm to about 40 ppm, from about 5 ppm to about 35 ppm or from about 10 ppm to about 35 ppm. The concentration of the sweetener comprising a sweetening amount of rebaudioside M is from about 100 ppm to about 600 ppm, more particularly from about 100 ppm to about 400 ppm or about 250 ppm to about 350 ppm.

In exemplary embodiments, the beverage comprising rebaudioside M and hesperetin dihydrochalcone has a sweetness of at least about 8 degrees Brix, more preferably about 9 degrees Brix, about 10 degrees Brix or about 11 degrees Brix.

In other exemplary embodiments, the sucrose equivalence of the beverage comprising rebaudioside M and hesperetin dihydrochalcone is enhanced by at least about 1.2-fold compared to the sucrose equivalence of a beverage without the hesperetin dihydrochalcone, more preferably at least about 1.5-fold, still more preferably about 2.0-fold.

In yet further exemplary embodiments, the Brix of the beverage comprising rebaudioside M and hesperetin dihydrochalcone is at least 2 degrees more than the Brix of the beverage without the hesperetin dihydrochalcone.

In still further exemplary embodiments, the beverage comprising rebaudioside M and hesperetin dihydrochalcone has at least one improved taste attribute compared to the taste attributes of a beverage without the hesperetin dihydrochalcone. For example, the beverage of the present invention exhibits one or more of the following: improved mouthfeel, a more sucrose-like taste, less sweetness linger, less licorice aftertaste, or less bitterness.

In a particular embodiment, the beverage is a diet beverage. In a more particular embodiment, the beverage is a diet carbonated beverage. In a still more particular embodiment, the beverage is a lemon-lime beverage.

In a particular embodiment, the present invention provides a beverage comprising hesperetin dihydrochalcone and a sweetener comprising a sweetening amount of siamenoside I. In exemplary embodiments, the beverage comprising siamenoside I and hesperetin dihydrochalcone has a sweetness of at least about 8 degrees Brix, more preferably about 9 degrees Brix, about 10 degrees Brix or about 11 degrees Brix. In other exemplary embodiments, the sucrose equivalence of the beverage comprising siamenoside I and hesperetin dihydrochalcone is enhanced by at least about 1.2-fold compared to the sucrose equivalence of a beverage without the hesperetin dihydrochalcone, more preferably at least about 1.5-fold, still more preferably about 2.0-fold. In yet further exemplary embodiments, the Brix of the beverage comprising siamenoside I and hesperetin dihydrochalcone is at least 1 degree more than the Brix of the beverage without the hesperetin dihydrochalcone, such as, for example, at least 2 degrees Brix. In still further exemplary embodiments, the beverage comprising siamenoside I and hesperetin dihydrochalcone has at least one improved taste attribute compared to the taste attributes of a beverage without the hesperetin dihydrochalcone. For example, the beverage of the present invention exhibits one or more of the following: improved mouthfeel, a more sucrose-like taste, less sweetness linger, less licorice aftertaste, or less bitterness.

In a particular embodiment, the present invention provides a beverage comprising hesperetin dihydrochalcone and a sweetener comprising a sweetening amount of the 1,6-α isomer of siamenoside I. In exemplary embodiments, the beverage comprising the 1,6-α isomer of siamenoside I and hesperetin dihydrochalcone has a sweetness of at least about 8 degrees Brix, more preferably about 9 degrees Brix, about 10 degrees Brix or about 11 degrees Brix. In other exemplary embodiments, the sucrose equivalence of the beverage comprising the 1,6-α isomer of siamenoside I and hesperetin dihydrochalcone is enhanced by at least about 1.2-fold compared to the sucrose equivalence of a beverage without the hesperetin dihydrochalcone, more preferably at least about 1.5-fold, still more preferably about 2.0-fold. In yet further exemplary embodiments, the Brix of the beverage comprising the 1,6-α isomer of siamenoside I and hesperetin dihydrochalcone is at least 1 degree more than the Brix of the beverage without the hesperetin dihydrochalcone, such as, for example, at least 2 degrees Brix. In still further exemplary embodiments, the beverage comprising the 1,6-α isomer of siamenoside I and hesperetin dihydrochalcone has at least one improved taste attribute compared to the taste attributes of a beverage without the hesperetin dihydrochalcone. For example, the beverage exhibits one or more of the following: improved mouthfeel, a more sucrose-like taste, less sweetness linger, less licorice aftertaste, or less bitterness.

In a particular embodiment, the present invention provides a beverage comprising hesperetin dihydrochalcone-4'-β-D-glucoside and a sweetener comprising a sweetening amount of rebaudioside M. In exemplary embodiments, the beverage comprising rebaudioside M and hesperetin dihydrochalcone-4'-β-D-glucoside has a sweetness of at least about 8 degrees Brix, more preferably about 9 degrees Brix, about 10 degrees Brix or about 11 degrees Brix. In other exemplary embodiments, the sucrose equivalence of the beverage comprising rebaudioside M and hesperetin dihydrochalcone-4'-β-D-glucoside is enhanced by at least about 1.2-fold compared to the sucrose equivalence of a beverage without the hesperetin dihydrochalcone-4'-β-D-glucoside, more preferably at least about 1.5-fold, still more preferably about 2.0-fold. In yet further exemplary embodiments, the Brix of the beverage comprising rebaudioside M and hesperetin dihydrochalcone-4'-β-D-glucoside is at least 1 degree more than the Brix of the beverage without the hesperetin dihydrochalcone-4'-β-D-glucoside, such as, for example, at least 2 degrees Brix. In still further exemplary embodiments, the beverage comprising rebaudioside M and hesperetin dihydrochalcone-4'-β-D-glucoside has at least one improved taste attribute compared to the taste attributes of a beverage without the hesperetin dihydrochalcone-4'-β-D-glucoside. For example, the beverage exhibits one or more of the following: improved mouthfeel, a more sucrose-like taste, less sweetness linger, less licorice aftertaste, or less bitterness.

In a particular embodiment, the present invention provides a beverage comprising hesperetin dihydrochalcone-4'-β-D-glucoside and a sweetener comprising a sweetening amount of siamenoside I. In exemplary embodiments, the beverage comprising siamenoside I and hesperetin dihydrochalcone-4'-β-D-glucoside has a sweetness of at least about 8 degrees Brix, more preferably about 9 degrees Brix, about 10 degrees Brix or about 11 degrees Brix. In other exemplary embodiments, the sucrose equivalence of the beverage comprising siamenoside I and hesperetin dihydrochalcone-4'-β-D-glucoside is enhanced by at least about 1.2-fold compared to the sucrose equivalence of a beverage without the hesperetin dihydrochalcone-4'-β-D-glucoside, more preferably at least about 1.5-fold, still more preferably about 2.0-fold. In yet further exemplary embodiments, the Brix of the beverage comprising siamenoside I and hesperetin dihydrochalcone-4'-β-D-glucoside is at least 1 degree more than the Brix of the beverage without the hesperetin dihydrochalcone-4'-β-D-glucoside, such as, for example, at least 2 degrees Brix. In still further exemplary embodiments, the beverage comprising siamenoside I and hesperetin dihydrochalcone-4'-β-D-glucoside has at least one improved taste attribute compared to the taste attributes of a beverage without the hesperetin dihydrochalcone-4'-β-D-glucoside. For example, the beverage exhibits one or more of the following: improved mouthfeel, a more sucrose-like taste, less sweetness linger, less licorice aftertaste, or less bitterness.

In a particular embodiment, the present invention provides a beverage comprising hesperetin dihydrochalcone-4'-β-D-glucoside and a sweetener comprising a sweetening amount of the 1,6-α-isomer of siamenoside I. In exemplary embodiments, the beverage comprising the 1,6-α-isomer of siamenoside I and hesperetin dihydrochalcone-4'-β-D-glucoside has a sweetness of at least about 8 degrees Brix, more preferably about 9 degrees Brix, about 10 degrees Brix or about 11 degrees Brix. In other exemplary embodiments, the sucrose equivalence of the beverage comprising the 1,6-α-isomer of siamenoside I and hesperetin dihydrochalcone-4'-β-D-glucoside is enhanced by at least about 1.2-fold compared to the sucrose equivalence of a beverage without the hesperetin dihydrochalcone-4'-β-D-glucoside, more preferably at least about 1.5-fold, still more preferably about 2.0-fold. In yet further exemplary embodiments, the Brix of the beverage comprising the 1,6-α-isomer of siamenoside I and hesperetin dihydrochalcone-4'-β-D-glucoside is at least 1 degree more than the Brix of the beverage without the hesperetin dihydrochalcone-4'-β-D-glucoside, such as, for example, at least 2 degrees Brix. In still further exemplary embodiments, the beverage comprising the 1,6-α-isomer of siamenoside I and hesperetin dihydrochalcone-4'-β-D-glucoside has at least one improved taste attribute compared to the taste attributes of a beverage without the hesperetin dihydrochalcone-4'-β-D-glucoside. For example, the beverage exhibits one or more of the following: improved mouthfeel, a more sucrose-like taste, less sweetness linger, less licorice aftertaste, or less bitterness.

**In** a particular embodiment, the beverage of the present invention is a flavored water beverage.

Other consumables include, but are not limited to, livestock products or seafood; processed meat products such as sausage and the like; retort food products, pickles, preserves boiled in soy sauce, delicacies, side dishes; soups; snacks such as potato chips, cookies, or the like; as shredded filler, leaf, stem, stalk, homogenized leaf cured and animal feed.

The consumable can optionally include additives, functional ingredients and combinations thereof, as described herein. Any of the additives and/or functional ingredients described above can be present in the consumable.

The consumable may further comprise one or more other additives, detailed herein below. In some embodiments, the sweetener composition contains additives including, but not limited to, carbohydrates, polyols, amino acids and their corresponding salts, poly-amino acids and their corresponding salts, sugar acids and their corresponding salts, nucleotides, organic acids, inorganic acids, organic salts including organic acid salts and organic base salts, inorganic salts, bitter compounds, flavorants and flavoring ingredients, astringent compounds, proteins or protein hydrolysates, surfactants, emulsifiers, weighing agents, gums, antioxidants, colorants, flavonoids, alcohols, polymers and combinations thereof. In some embodiments, the additives act to improve the temporal and flavor profile to provide a taste similar to sucrose.

In one embodiment, the consumable further comprises one or more polyols. The term "polyol", as used herein, refers to a molecule that contains more than one hydroxyl group. A polyol may be a diol, triol, or a tetraol which contains 2, 3, and 4 hydroxyl groups respectively. A polyol also may contain more than 4 hydroxyl groups, such as a pentaol, hexaol, heptaol, or the like, which contain 5, 6, or 7 hydroxyl groups, respectively. Additionally, a polyol also may be a sugar alcohol, polyhydric alcohol, or polyalcohol which is a reduced form of carbohydrate, wherein the carbonyl group (aldehyde or ketone, reducing sugar) has been reduced to a primary or secondary hydroxyl group.

Non-limiting examples of polyols in some embodiments include maltitol, mannitol, sorbitol, lactitol, xylitol, isomalt, propylene glycol, glycerol (glycerin), threitol, galactitol, palatinose, reduced isomalto-oligosaccharides, reduced xylo-oligosaccharides, reduced gentio-oligosaccharides, reduced maltose syrup, reduced glucose syrup, and sugar alcohols or any other carbohydrates capable of being reduced which do not adversely affect taste.

Suitable amino acid additives include, but are not limited to, aspartic acid, arginine, glycine, glutamic acid, proline, threonine, theanine, cysteine, cystine, alanine, valine, tyrosine, leucine, arabinose, trans-4-hydroxyproline, isoleucine, asparagine, serine, lysine, histidine, ornithine, methionine, carnitine, aminobutyric acid (α-, β-, and/or δ-isomers), glutamine, hydroxyproline, taurine, norvaline, sarcosine, and their salt forms such as sodium or potassium salts or acid salts. The amino acid additives also may be in the D- or L-configuration and in the mono-, di-, or tri-form of the same or different amino acids. Additionally, the amino acids may be α-, β-, γ- and/or δ-isomers if appropriate. Combinations of the foregoing amino acids and their corresponding salts (*e.g.*, sodium, potassium, calcium, magnesium salts or other alkali or alkaline earth metal salts thereof, or acid salts) also are suitable additives in some embodiments. The amino acids may be natural or synthetic. The amino acids also may be modified. Modified amino acids refers to any amino acid wherein at least one atom has been added, removed, substituted, or combinations thereof (*e.g.*, N-alkyl amino acid, N-acyl amino acid, or N-methyl amino acid). Non-limiting examples of modified amino acids include amino acid derivatives such as trimethyl glycine, N-methyl-glycine, and N-methyl-alanine. As used herein, modified amino acids encompass both modified and unmodified amino acids. As used herein, amino acids also encompass both peptides and polypeptides (*e.g.*, dipeptides, tripeptides, tetrapeptides, and pentapeptides) such as glutathione and L-alanyl-L-glutamine. Suitable polyamino acid additives include poly-L-aspartic acid, poly-L-lysine (e.g., poly-L-α-lysine or poly-L-ε-lysine), poly-L-ornithine (e.g., poly-L-α-ornithine or poly-L-ε-ornithine), poly-L-arginine, other polymeric forms of amino acids, and salt forms thereof (*e.g.*, calcium, potassium, sodium, or magnesium salts such as L-glutamic acid mono sodium salt). The poly-amino acid additives also may be in the D- or L-configuration. Additionally, the poly-amino acids may be α-, β-, γ-, δ-, and ε-isomers if appropriate. Combinations of the foregoing poly-amino acids and their corresponding salts (*e.g.*, sodium, potassium, calcium, magnesium salts or other alkali or alkaline earth metal salts thereof or acid salts) also are suitable additives in some embodiments. The poly-amino acids described herein also may comprise co-polymers of different amino acids. The poly-amino acids may be natural or synthetic. The poly-amino acids also may be modified, such that at least one atom has been added, removed, substituted, or combinations thereof (e.g., N-alkyl poly-amino acid or N-acyl poly-amino acid). As used herein, poly-amino acids encompass both modified and unmodified poly-amino acids. For example, modified poly-amino acids include, but are not limited to, poly-amino acids of various molecular weights (MW), such as poly-L-α-lysine with a MW of 1,500, MW of 6,000, MW of 25,200, MW of 63,000, MW of 83,000, or MW of 300,000.

In particular embodiments, the amino acid is present in the consumable in an amount from about 10 ppm to about 50,000 ppm. In another embodiment, the amino acid is present in the consumable in an amount from about 1,000 ppm to about 10,000 ppm, such as, for example, from about 2,500 ppm to about 5,000 ppm or from about 250 ppm to about 7,500 ppm.

Suitable sugar acid additives include, but are not limited to, aldonic, uronic, aldaric, alginic, gluconic, glucuronic, glucaric, galactaric, galacturonic, and salts thereof (e.g., sodium, potassium, calcium, magnesium salts or other physiologically acceptable salts), and combinations thereof.

Suitable nucleotide additives include, but are not limited to, inosine monophosphate ("IMP"), guanosine monophosphate ("GMP"), adenosine monophosphate ("AMP"), cytosine monophosphate (CMP), uracil monophosphate (UMP), inosine diphosphate, guanosine diphosphate, adenosine diphosphate, cytosine diphosphate, uracil diphosphate, inosine triphosphate, guanosine triphosphate, adenosine triphosphate, cytosine triphosphate, uracil triphosphate, alkali or alkaline earth metal salts thereof, and combinations thereof. The nucleotides described herein also may comprise nucleotide-related additives, such as nucleosides or nucleic acid bases (*e.g.*, guanine, cytosine, adenine, thymine, uracil).

Suitable organic acid additives include any compound which comprises a -COOH moiety, such as, for example, C2-C30 carboxylic acids, substituted hydroxyl C2-C30 carboxylic acids, butyric acid (ethyl esters), substituted butyric acid (ethyl esters), benzoic acid, substituted benzoic acids (*e.g.*, 2,4-dihydroxybenzoic acid), substituted cinnamic acids, hydroxyacids, substituted hydroxybenzoic acids, anisic acid substituted cyclohexyl carboxylic acids, tannic acid, aconitic acid, lactic acid, tartaric acid, citric acid, isocitric acid, gluconic acid, glucoheptonic acids, adipic acid, hydroxycitric acid, malic acid, fruitaric acid (a blend of malic, fumaric, and tartaric acids), fumaric acid, maleic acid, succinic acid, chlorogenic acid, salicylic acid, creatine, caffeic acid, bile acids, acetic acid, ascorbic acid, alginic acid, erythorbic acid, polyglutamic acid, glucono delta lactone, and their alkali or alkaline earth metal salt derivatives thereof. In addition, the organic acid additives also may be in either the D- or L-configuration.

Suitable organic acid additive salts include, but are not limited to, sodium, calcium, potassium, and magnesium salts of all organic acids, such as salts of citric acid, malic acid, tartaric acid, fumaric acid, lactic acid (*e.g.,* sodium lactate), alginic acid (*e.g.,* sodium alginate), ascorbic acid (*e.g.,* sodium ascorbate), benzoic acid (*e.g.,* sodium benzoate or potassium benzoate), sorbic acid and adipic acid. The examples of the organic acid additives described optionally may be substituted with at least one group chosen from hydrogen, alkyl, alkenyl, alkynyl, halo, haloalkyl, carboxyl, acyl, acyloxy, amino, amido, carboxyl derivatives, alkylamino, dialkylamino, arylamino, alkoxy, aryloxy, nitro, cyano, sulfo, thiol, imine, sulfonyl, sulfenyl, sulfinyl, sulfamyl, carboxalkoxy, carboxamido, phosphonyl, phosphinyl, phosphoryl, phosphino, thioester, thioether, anhydride, oximino, hydrazino, carbamyl, phosphor or phosphonato. In particular embodiments, the organic acid additive is present in the sweetener composition in an amount effective to provide a concentration from about 10 ppm to about 5,000 ppm when present in a consumable, such as, for example, a beverage.

Suitable inorganic acid additives include, but are not limited to, phosphoric acid, phosphorous acid, polyphosphoric acid, hydrochloric acid, sulfuric acid, carbonic acid, sodium dihydrogen phosphate, and alkali or alkaline earth metal salts thereof (*e.g.*, inositol hexaphosphate Mg/Ca).

The inorganic acid additive is present in the consumable in a concentration from about 25 ppm to about 25,000 ppm.

Suitable bitter compound additives include, but are not limited to, caffeine, quinine, urea, bitter orange oil, naringin, quassia, and salts thereof.

The bitter compound is present in the consumable in a concentration from about 25 ppm to about 25,000 ppm.

Suitable flavorants and flavoring ingredient additives include, but are not limited to, vanillin, vanilla extract, mango extract, cinnamon, citrus, coconut, ginger, viridiflorol, almond, menthol (including menthol without mint), grape skin extract, and grape seed extract. "Flavorant" and "flavoring ingredient" are synonymous and can include natural or synthetic substances or combinations thereof. Flavorants also include any other substance which imparts flavor and may include natural or non-natural (synthetic) substances which are safe for human or animals when used in a generally accepted range. Non-limiting examples of proprietary flavorants include Döhler^{™} Natural Flavoring Sweetness Enhancer K14323 (Döhler^{™}, Darmstadt, Germany), Symrise^{™} Natural Flavor Mask for Sweeteners 161453 and 164126 (Symrise^{™}, Holzminden, Germany), Natural Advantage^{™} Bitterness Blockers 1, 2, 9 and 10 (Natural Advantage^{™}, Freehold, New Jersey, U.S.A.), and Sucramask^{™} (Creative Research Management, Stockton, California, U.S.A.).

The flavorant is present in the consumable in a concentration from about 0.1 ppm to about 4,000 ppm.

Suitable polymer additives include, but are not limited to, chitosan, pectin, pectic, pectinic, polyuronic, polygalacturonic acid, starch, food hydrocolloid or crude extracts thereof (e.g., gum acacia senegal (Fibergum^{™}), gum acacia seyal, carageenan), poly-L-lysine (e.g., poly-L-α-lysine or poly-L-ε-lysine), poly-L-ornithine (e.g., poly-L-α-ornithine or poly-L-ε-ornithine), polypropylene glycol, polyethylene glycol, poly(ethylene glycol methyl ether), polyarginine, polyaspartic acid, polyglutamic acid, polyethylene imine, alginic acid, sodium alginate, propylene glycol alginate, and sodium polyethyleneglycolalginate, sodium hexametaphosphate and its salts, and other cationic polymers and anionic polymers.

The polymer is present in the consumable a concentration from about 30 ppm to about 2,000 ppm.

Suitable protein or protein hydrolysate additives include, but are not limited to, bovine serum albumin (BSA), whey protein (including fractions or concentrates thereof such as 90% instant whey protein isolate, 34% whey protein, 50% hydrolyzed whey protein, and 80% whey protein concentrate), soluble rice protein, soy protein, protein isolates, protein hydrolysates, reaction products of protein hydrolysates, glycoproteins, and/or proteoglycans containing amino acids (e.g., glycine, alanine, serine, threonine, asparagine, glutamine, arginine, valine, isoleucine, leucine, norvaline, methionine, proline, tyrosine, hydroxyproline, and the like), collagen (e.g., gelatin), partially hydrolyzed collagen (e.g., hydrolyzed fish collagen), and collagen hydrolysates (e.g., porcine collagen hydrolysate).

The protein hydrolysate is present in the consumable in a concentration from about 200 ppm to about 50,000 ppm.

Suitable surfactant additives include, but are not limited to, polysorbates (e.g., polyoxyethylene sorbitan monooleate (polysorbate 80), polysorbate 20, polysorbate 60), sodium dodecylbenzenesulfonate, dioctyl sulfosuccinate or dioctyl sulfosuccinate sodium, sodium dodecyl sulfate, cetylpyridinium chloride (hexadecylpyridinium chloride), hexadecyltrimethylammonium bromide, sodium cholate, carbamoyl, choline chloride, sodium glycocholate, sodium taurodeoxycholate, lauric arginate, sodium stearoyl lactylate, sodium taurocholate, lecithins, sucrose oleate esters, sucrose stearate esters, sucrose palmitate esters, sucrose laurate esters, and other emulsifiers, and the like.

The surfactant additive is present in the consumable in a concentration from about 30 ppm to about 2,000 ppm.

Suitable flavonoid additives are classified as flavonols, flavones, flavanones, flavan-3-ols, isoflavones, or anthocyanidins. Non-limiting examples of flavonoid additives include, but are not limited to, catechins (e.g., green tea extracts such as Polyphenon^{™} 60, Polyphenon^{™} 30, and Polyphenon^{™} 25 (Mitsui Norin Co., Ltd., Japan), polyphenols, rutins (e.g., enzyme modified rutin Sanmelin^{™} AO (San-fi Gen F.F.I., Inc., Osaka, Japan)), neohesperidin, naringin, neohesperidin dihydrochalcone, and the like.

The flavonoid additive is present in the consumable in a concentration from about 0.1 ppm to about 1,000 ppm.

Suitable alcohol additives include, but are not limited to, ethanol. In particular embodiments, the alcohol additive is present in the consumable in a concentration from about 625 ppm to about 10,000 ppm.

Suitable astringent compound additives include, but are not limited to, tannic acid, europium chloride (EuCl₃), gadolinium chloride (GdCl₃), terbium chloride (TbCl₃), alum, tannic acid, and polyphenols (e.g., tea polyphenols). The astringent additive is present in the consumable in a concentration from about 10 ppm to about 5,000 ppm.

### Functional Ingredients

The consumables provided herein can also contain one or more functional ingredients, which provide a real or perceived heath benefit to the composition. Functional ingredients include, but are not limited to, saponins, antioxidants, dietary fiber sources, fatty acids, vitamins, glucosamine, minerals, preservatives, hydration agents, probiotics, prebiotics, weight management agents, osteoporosis management agents, phytoestrogens, long chain primary aliphatic saturated alcohols, phytosterols and combinations thereof.

### Saponin

In certain embodiments, the functional ingredient is at least one saponin. As used herein, the at least one saponin may comprise a single saponin or a plurality of saponins as a functional ingredient for the composition provided herein. Generally, according to particular embodiments of this invention, the at least one saponin is present in the composition in an amount sufficient to promote health and wellness.

Saponins are glycosidic natural plant products comprising an aglycone ring structure and one or more sugar moieties. The combination of the nonpolar aglycone and the water soluble sugar moiety gives saponins surfactant properties, which allow them to form a foam when shaken in an aqueous solution.

The saponins are grouped together based on several common properties. In particular, saponins are surfactants which display hemolytic activity and form complexes with cholesterol. Although saponins share these properties, they are structurally diverse. The types of aglycone ring structures forming the ring structure in saponins can vary greatly. Non-limiting examples of the types of aglycone ring structures in saponin for use in particular embodiments of the invention include steroids, triterpenoids, and steroidal alkaloids. Non-limiting examples of specific aglycone ring structures for use in particular embodiments of the invention include soyasapogenol A, soyasapogenol B and soyasopogenol E. The number and type of sugar moieties attached to the aglycone ring structure can also vary greatly. Non-limiting examples of sugar moieties for use in particular embodiments of the invention include glucose, galactose, glucuronic acid, xylose, rhamnose, and methylpentose moieties. Non-limiting examples of specific saponins for use in particular embodiments of the invention include group A acetyl saponin, group B acetyl saponin, and group E acetyl saponin.

Saponins can be found in a large variety of plants and plant products, and are especially prevalent in plant skins and barks where they form a waxy protective coating. Several common sources of saponins include soybeans, which have approximately 5% saponin content by dry weight, soapwort plants (*Saponaria*), the root of which was used historically as soap, as well as alfalfa, aloe, asparagus, grapes, chickpeas, yucca, and various other beans and weeds. Saponins may be obtained from these sources by using extraction techniques well known to those of ordinary skill in the art. A description of conventional extraction techniques can be found in U.S. Pat. Appl. No. 2005/0123662.

### Antioxidant

In certain embodiments, the functional ingredient is at least one antioxidant. As used herein, the at least one antioxidant may comprise a single antioxidant or a plurality of antioxidants as a functional ingredient for the compositions provided herein. Generally, according to particular embodiments of this invention, the at least one antioxidant is present in the composition in an amount sufficient to promote health and wellness.

As used herein "antioxidant" refers to any substance which inhibits, suppresses, or reduces oxidative damage to cells and biomolecules. Without being bound by theory, it is believed that antioxidants inhibit, suppress, or reduce oxidative damage to cells or biomolecules by stabilizing free radicals before they can cause harmful reactions. As such, antioxidants may prevent or postpone the onset of some degenerative diseases.

Examples of suitable antioxidants for embodiments of this invention include, but are not limited to, vitamins, vitamin cofactors, minerals, hormones, carotenoids, carotenoid terpenoids, non-carotenoid terpenoids, flavonoids, flavonoid polyphenolics (e.g., bioflavonoids), flavonols, flavones, phenols, polyphenols, esters of phenols, esters of polyphenols, nonflavonoid phenolics, isothiocyanates, and combinations thereof. In some embodiments, the antioxidant is vitamin A, vitamin C, vitamin E, ubiquinone, mineral selenium, manganese, melatonin, α-carotene, β-carotene, lycopene, lutein, zeanthin, crypoxanthin, reservatol, eugenol, quercetin, catechin, gossypol, hesperetin, curcumin, ferulic acid, thymol, hydroxytyrosol, tumeric, thyme, olive oil, lipoic acid, glutathinone, gutamine, oxalic acid, tocopherol-derived compounds, butylated hydroxyanisole (BHA), butylated hydroxytoluene (BHT), ethylenediaminetetraacetic acid (EDTA), tert-butylhydroquinone, acetic acid, pectin, tocotrienol, tocopherol, coenzyme Q10, zeaxanthin, astaxanthin, canthaxantin, saponins, limonoids, kaempfedrol, myricetin, isorhamnetin, proanthocyanidins, quercetin, rutin, luteolin, apigenin, tangeritin, hesperetin, naringenin, erodictyol, flavan-3-ols (e.g., anthocyanidins), gallocatechins, epicatechin and its gallate forms, epigallocatechin and its gallate forms (ECGC) theaflavin and its gallate forms, thearubigins, isoflavone, phytoestrogens, genistein, daidzein, glycitein, anythocyanins, cyaniding, delphinidin, malvidin, pelargonidin, peonidin, petunidin, ellagic acid, gallic acid, salicylic acid, rosmarinic acid, cinnamic acid and its derivatives (e.g., ferulic acid), chlorogenic acid, chicoric acid, gallotannins, ellagitannins, anthoxanthins, betacyanins and other plant pigments, silymarin, citric acid, lignan, antinutrients, bilirubin, uric acid, R-α-lipoic acid, N-acetylcysteine, emblicanin, apple extract, apple skin extract (applephenon), rooibos extract red, rooibos extract, green, hawthorn berry extract, red raspberry extract, green coffee antioxidant (GCA), aronia extract 20%, grape seed extract (VinOseed), cocoa extract, hops extract, mangosteen extract, mangosteen hull extract, cranberry extract, pomegranate extract, pomegranate hull extract, pomegranate seed extract, hawthorn berry extract, pomella pomegranate extract, cinnamon bark extract, grape skin extract, bilberry extract, pine bark extract, pycnogenol, elderberry extract, mulberry root extract, wolfberry (gogi) extract, blackberry extract, blueberry extract, blueberry leaf extract, raspberry extract, turmeric extract, citrus bioflavonoids, black currant, ginger, acai powder, green coffee bean extract, green tea extract, and phytic acid, or combinations thereof. In alternate embodiments, the antioxidant is a synthetic antioxidant such as butylated hydroxytolune or butylated hydroxyanisole, for example. Other sources of suitable antioxidants for embodiments of this invention include, but are not limited to, fruits, vegetables, tea, cocoa, chocolate, spices, herbs, rice, organ meats from livestock, yeast, whole grains, or cereal grains.

Particular antioxidants belong to the class of phytonutrients called polyphenols (also known as "polyphenolics"), which are a group of chemical substances found in plants, characterized by the presence of more than one phenol group per molecule. A variety of health benefits may be derived from polyphenols, including prevention of cancer, heart disease, and chronic inflammatory disease and improved mental strength and physical strength, for example. Suitable polyphenols for embodiments of this invention include catechins, proanthocyanidins, procyanidins, anthocyanins, quercerin, rutin, reservatrol, isoflavones, curcumin, punicalagin, ellagitannin, hesperidin, naringin, citrus flavonoids, chlorogenic acid, other similar materials, and combinations thereof.

In particular embodiments, the antioxidant is a catechin such as, for example, epigallocatechin gallate (EGCG). Suitable sources of catechins for embodiments of this invention include, but are not limited to, green tea, white tea, black tea, oolong tea, chocolate, cocoa, red wine, grape seed, red grape skin, purple grape skin, red grape juice, purple grape juice, berries, pycnogenol, and red apple peel.

In some embodiments, the antioxidant is chosen from proanthocyanidins, procyanidins or combinations thereof. Suitable sources of proanthocyanidins and procyanidins for embodiments of this invention include, but are not limited to, red grapes, purple grapes, cocoa, chocolate, grape seeds, red wine, cacao beans, cranberry, apple peel, plum, blueberry, black currants, choke berry, green tea, sorghum, cinnamon, barley, red kidney bean, pinto bean, hops, almonds, hazelnuts, pecans, pistachio, pycnogenol, and colorful berries.

In particular embodiments, the antioxidant is an anthocyanin. Suitable sources of anthocyanins for embodiments of this invention include, but are not limited to, red berries, blueberries, bilberry, cranberry, raspberry, cherry, pomegranate, strawberry, elderberry, choke berry, red grape skin, purple grape skin, grape seed, red wine, black currant, red currant, cocoa, plum, apple peel, peach, red pear, red cabbage, red onion, red orange, and blackberries.

In some embodiments, the antioxidant is chosen from quercetin, rutin or combinations thereof. Suitable sources of quercetin and rutin for embodiments of this invention include, but are not limited to, red apples, onions, kale, bog whortleberry, lingonberrys, chokeberry, cranberry, blackberry, blueberry, strawberry, raspberry, black currant, green tea, black tea, plum, apricot, parsley, leek, broccoli, chili pepper, berry wine, and ginkgo.

In some embodiments, the antioxidant is reservatrol. Suitable sources of reservatrol for embodiments of this invention include, but are not limited to, red grapes, peanuts, cranberry, blueberry, bilberry, mulberry, Japanese Itadori tea, and red wine.

In particular embodiments, the antioxidant is an isoflavone. Suitable sources of isoflavones for embodiments of this invention include, but are not limited to, soy beans, soy products, legumes, alfalfa sprouts, chickpeas, peanuts, and red clover.

In some embodiments, the antioxidant is curcumin. Suitable sources of curcumin for embodiments of this invention include, but are not limited to, turmeric and mustard.

In particular embodiments, the antioxidant is chosen from punicalagin, ellagitannin or combinations thereof. Suitable sources of punicalagin and ellagitannin for embodiments of this invention include, but are not limited to, pomegranate, raspberry, strawberry, walnut, and oak-aged red wine.

In particular embodiments, the antioxidant is chlorogenic acid. Suitable sources of chlorogenic acid for embodiments of this invention include, but are not limited to, green coffee, yerba mate, red wine, grape seed, red grape skin, purple grape skin, red grape juice, purple grape juice, apple juice, cranberry, pomegranate, blueberry, strawberry, sunflower, Echinacea, pycnogenol, and apple peel.

### Dietary Fiber

In certain embodiments, the functional ingredient is at least one dietary fiber source. As used herein, the at least one dietary fiber source may comprise a single dietary fiber source or a plurality of dietary fiber sources as a functional ingredient for the compositions provided herein. Generally, according to particular embodiments of this invention, the at least one dietary fiber source is present in the composition in an amount sufficient to promote health and wellness.

Numerous polymeric carbohydrates having significantly different structures in both composition and linkages fall within the definition of dietary fiber. Such compounds are well known to those skilled in the art, non-limiting examples of which include non-starch polysaccharides, lignin, cellulose, methylcellulose, the hemicelluloses, β-glucans, pectins, gums, mucilage, waxes, inulins, oligosaccharides, fructooligosaccharides, cyclodextrins, chitins, and combinations thereof.

Polysaccharides are complex carbohydrates composed of monosaccharides joined by glycosidic linkages. Non-starch polysaccharides are bonded with β-linkages, which humans are unable to digest due to a lack of an enzyme to break the β-linkages. Conversely, digestible starch polysaccharides generally comprise α(1-4) linkages.

Lignin is a large, highly branched and cross-linked polymer based on oxygenated phenylpropane units. Cellulose is a linear polymer of glucose molecules joined by a β(1-4) linkage, which mammalian amylases are unable to hydrolyze. Methylcellulose is a methyl ester of cellulose that is often used in foodstuffs as a thickener, and emulsifier. It is commercially available (e.g., Citrucel by GlaxoSmithKline, Celevac by Shire Pharmaceuticals). Hemicelluloses are highly branched polymers consisting mainly of glucurono- and 4-O-methylglucuroxylans. β-Glucans are mixed-linkage (1-3), (1-4) β-D-glucose polymers found primarily in cereals, such as oats and barley. Pectins, such as beta pectin, are a group of polysaccharides composed primarily of D-galacturonic acid, which is methoxylated to variable degrees.

Gums and mucilages represent a broad array of different branched structures. Guar gum, derived from the ground endosperm of the guar seed, is a galactomannan. Guar gum is commercially available (e.g., Benefiber by Novartis AG). Other gums, such as gum arabic and pectins, have still different structures. Still other gums include xanthan gum, gellan gum, tara gum, psylium seed husk gum, and locust been gum.

Waxes are esters of ethylene glycol and two fatty acids, generally occurring as a hydrophobic liquid that is insoluble in water.

Inulins comprise naturally occurring oligosaccharides belonging to a class of carbohydrates known as fructans. They generally are comprised of fructose units joined by β(2-1) glycosidic linkages with a terminal glucose unit. Oligosaccharides are saccharide polymers containing typically three to six component sugars. They are generally found either O- or N-linked to compatible amino acid side chains in proteins or to lipid molecules. Fructooligosaccharides are oligosaccharides consisting of short chains of fructose molecules.

Food sources of dietary fiber include, but are not limited to, grains, legumes, fruits, and vegetables. Grains providing dietary fiber include, but are not limited to, oats, rye, barley, wheat,. Legumes providing fiber include, but are not limited to, peas and beans such as soybeans. Fruits and vegetables providing a source of fiber include, but are not limited to, apples, oranges, pears, bananas, berries, tomatoes, green beans, broccoli, cauliflower, carrots, potatoes, celery. Plant foods such as bran, nuts, and seeds (such as flax seeds) are also sources of dietary fiber. Parts of plants providing dietary fiber include, but are not limited to, the stems, roots, leaves, seeds, pulp, and skin.

Although dietary fiber generally is derived from plant sources, indigestible animal products such as chitins are also classified as dietary fiber. Chitin is a polysaccharide composed of units of acetylglucosamine joined by β(1-4) linkages, similar to the linkages of cellulose.

Sources of dietary fiber often are divided into categories of soluble and insoluble fiber based on their solubility in water. Both soluble and insoluble fibers are found in plant foods to varying degrees depending upon the characteristics of the plant. Although insoluble in water, insoluble fiber has passive hydrophilic properties that help increase bulk, soften stools, and shorten transit time of fecal solids through the intestinal tract.

Unlike insoluble fiber, soluble fiber readily dissolves in water. Soluble fiber undergoes active metabolic processing via fermentation in the colon, increasing the colonic microflora and thereby increasing the mass of fecal solids. Fermentation of fibers by colonic bacteria also yields end-products with significant health benefits. For example, fermentation of the food masses produces gases and short-chain fatty acids. Acids produced during fermentation include butyric, acetic, propionic, and valeric acids that have various beneficial properties such as stabilizing blood glucose levels by acting on pancreatic insulin release and providing liver control by glycogen breakdown. In addition, fiber fermentation may reduce atherosclerosis by lowering cholesterol synthesis by the liver and reducing blood levels of LDL and triglycerides. The acids produced during fermentation lower colonic pH, thereby protecting the colon lining from cancer polyp formation. The lower colonic pH also increases mineral absorption, improves the barrier properties of the colonic mucosal layer, and inhibits inflammatory and adhesion irritants. Fermentation of fibers also may benefit the immune system by stimulating production of T-helper cells, antibodies, leukocytes, splenocytes, cytokinins and lymphocytes.

### Fatty Acid

In certain embodiments, the functional ingredient is at least one fatty acid. As used herein, the at least one fatty acid may be single fatty acid or a plurality of fatty acids as a functional ingredient for the compositions provided herein. Generally, according to particular embodiments of this invention, the at least one fatty acid is present in the composition in an amount sufficient to promote health and wellness.

As used herein, "fatty acid" refers to any straight chain monocarboxylic acid and includes saturated fatty acids, unsaturated fatty acids, long chain fatty acids, medium chain fatty acids, short chain fatty acids, fatty acid precursors (including omega-9 fatty acid precursors), and esterified fatty acids. As used herein, "long chain polyunsaturated fatty acid" refers to any polyunsaturated carboxylic acid or organic acid with a long aliphatic tail. As used herein, "omega-3 fatty acid" refers to any polyunsaturated fatty acid having a first double bond as the third carbon-carbon bond from the terminal methyl end of its carbon chain. In particular embodiments, the omega-3 fatty acid may comprise a long chain omega-3 fatty acid. As used herein, "omega-6 fatty acid" any polyunsaturated fatty acid having a first double bond as the sixth carbon-carbon bond from the terminal methyl end of its carbon chain.

Suitable omega-3 fatty acids for use in embodiments of the present invention can be derived from algae, fish, animals, plants, or combinations thereof, for example. Examples of suitable omega-3 fatty acids include, but are not limited to, linolenic acid, alpha-linolenic acid, eicosapentaenoic acid, docosahexaenoic acid, stearidonic acid, eicosatetraenoic acid and combinations thereof. In some embodiments, suitable omega-3 fatty acids can be provided in fish oils, (e.g., menhaden oil, tuna oil, salmon oil, bonito oil, and cod oil), microalgae omega-3 oils or combinations thereof. In particular embodiments, suitable omega-3 fatty acids may be derived from commercially available omega-3 fatty acid oils such as Microalgae DHA oil (from Martek, Columbia, MD), OmegaPure (from Omega Protein, Houston, TX), Marinol C-38 (from Lipid Nutrition, Channahon, IL), Bonito oil and MEG-3 (from Ocean Nutrition, Dartmouth, NS), Evogel (from Symrise, Holzminden, Germany), Marine Oil, from tuna or salmon (from Arista Wilton, CT), OmegaSource 2000, Marine Oil, from menhaden and Marine Oil, from cod (from OmegaSource, RTP, NC).

Suitable omega-6 fatty acids include, but are not limited to, linoleic acid, gamma-linolenic acid, dihommo-gamma-linolenic acid, arachidonic acid, eicosadienoic acid, docosadienoic acid, adrenic acid, docosapentaenoic acid and combinations thereof.

Suitable esterified fatty acids for embodiments of the present invention may include, but are not limited to, monoacylgycerols containing omega-3 and/or omega-6 fatty acids, diacylgycerols containing omega-3 and/or omega-6 fatty acids, or triacylgycerols containing omega-3 and/or omega-6 fatty acids and combinations thereof.

### Vitamin

In certain embodiments, the functional ingredient is at least one vitamin.

As used herein, the at least one vitamin may be single vitamin or a plurality of vitamins as a functional ingredient for the compositions provided herein. Generally, according to particular embodiments of this invention, the at least one vitamin is present in the composition in an amount sufficient to promote health and wellness.

Vitamins are organic compounds that the human body needs in small quantities for normal functioning. The body uses vitamins without breaking them down, unlike other nutrients such as carbohydrates and proteins. To date, thirteen vitamins have been recognized, and one or more can be used in the compositions herein. Suitable vitamins include, vitamin A, vitamin D, vitamin E, vitamin K, vitamin B1, vitamin B2, vitamin B3, vitamin B5, vitamin B6, vitamin B7, vitamin B9, vitamin B12, and vitamin C.

Various other compounds have been classified as vitamins by some authorities. These compounds may be termed pseudo-vitamins and include, but are not limited to, compounds such as ubiquinone (coenzyme Q10), pangamic acid, dimethylglycine, taestrile, amygdaline, flavanoids, para-aminobenzoic acid, adenine, adenylic acid, and s-methylmethionine. As used herein, the term vitamin includes pseudo-vitamins.

In some embodiments, the vitamin is a fat-soluble vitamin chosen from vitamin A, D, E, K and combinations thereof.

In other embodiments, the vitamin is a water-soluble vitamin chosen from vitamin B1, vitamin B2, vitamin B3, vitamin B6, vitamin B12, folic acid, biotin, pantothenic acid, vitamin C and combinations thereof.

### Glucosamine

In certain embodiments, the functional ingredient is glucosamine.

Generally, according to particular embodiments of this invention, glucosamine is present in the compositions in an amount sufficient to promote health and wellness.

Glucosamine, also called chitosamine, is an amino sugar that is believed to be an important precursor in the biochemical synthesis of glycosylated proteins and lipids. D-glucosamine occurs naturally in the cartilage in the form of glucosamine-6-phosphate, which is synthesized from fructose-6-phosphate and glutamine. However, glucosamine also is available in other forms, non-limiting examples of which include glucosamine hydrochloride, glucosamine sulfate, N-acetyl-glucosamine, or any other salt forms or combinations thereof. Glucosamine may be obtained by acid hydrolysis of the shells of lobsters, crabs, shrimps, or prawns using methods well known to those of ordinary skill in the art. In a particular embodiment, glucosamine may be derived from fungal biomass containing chitin, as described in U.S. Patent Publication No. 2006/0172392.

The compositions can further comprise chondroitin sulfate.

### Mineral

In certain embodiments, the functional ingredient is at least one mineral.

As used herein, the at least one mineral may be single mineral or a plurality of minerals as a functional ingredient for the compositions provided herein. Generally, according to particular embodiments of this invention, the at least one mineral is present in the composition in an amount sufficient to promote health and wellness.

Minerals, in accordance with the teachings of this invention, comprise inorganic chemical elements required by living organisms. Minerals are comprised of a broad range of compositions (e.g., elements, simple salts, and complex silicates) and also vary broadly in crystalline structure. They may naturally occur in foods and beverages, may be added as a supplement, or may be consumed or administered separately from foods or beverages.

Minerals may be categorized as either bulk minerals, which are required in relatively large amounts, or trace minerals, which are required in relatively small amounts. Bulk minerals generally are required in amounts greater than or equal to about 100 mg per day and trace minerals are those that are required in amounts less than about 100 mg per day.

In particular embodiments of this invention, the mineral is chosen from bulk minerals, trace minerals or combinations thereof. Non-limiting examples of bulk minerals include calcium, chlorine, magnesium, phosphorous, potassium, sodium, and sulfur. Non-limiting examples of trace minerals include chromium, cobalt, copper, fluorine, iron, manganese, molybdenum, selenium, zinc, and iodine. Although iodine generally is classified as a trace mineral, it is required in larger quantities than other trace minerals and often is categorized as a bulk mineral.

In other particular embodiments of this invention, the mineral is a trace mineral, believed to be necessary for human nutrition, non-limiting examples of which include bismuth, boron, lithium, nickel, rubidium, silicon, strontium, tellurium, tin, titanium, tungsten, and vanadium.

The minerals embodied herein may be in any form known to those of ordinary skill in the art. For example, in a particular embodiment the minerals may be in their ionic form, having either a positive or negative charge. In another particular embodiment the minerals may be in their molecular form. For example, sulfur and phosphorous often are found naturally as sulfates, sulfides, and phosphates.

### Preservative

In certain embodiments, the functional ingredient is at least one preservative.

As used herein, the at least one preservative may be single preservative or a plurality of preservatives as a functional ingredient for the compositions provided herein. Generally, according to particular embodiments of this invention, the at least one preservative is present in the composition in an amount sufficient to promote health and wellness.

In particular embodiments of this invention, the preservative is chosen from antimicrobials, antioxidants, antienzymatics or combinations thereof. Non-limiting examples of antimicrobials include sulfites, propionates, benzoates, sorbates, nitrates, nitrites, bacteriocins, salts, sugars, acetic acid, dimethyl dicarbonate (DMDC), ethanol, and ozone.

According to a particular embodiment, the preservative is a sulfite. Sulfites include, but are not limited to, sulfur dioxide, sodium bisulfite, and potassium hydrogen sulfite.

According to another particular embodiment, the preservative is a propionate. Propionates include, but are not limited to, propionic acid, calcium propionate, and sodium propionate.

According to yet another particular embodiment, the preservative is a benzoate. Benzoates include, but are not limited to, sodium benzoate and benzoic acid.

In another particular embodiment, the preservative is a sorbate. Sorbates include, but are not limited to, potassium sorbate, sodium sorbate, calcium sorbate, and sorbic acid.

In still another particular embodiment, the preservative is a nitrate and/or a nitrite. Nitrates and nitrites include, but are not limited to, sodium nitrate and sodium nitrite.

In yet another particular embodiment, the at least one preservative is a bacteriocin, such as, for example, nisin.

In another particular embodiment, the preservative is ethanol.

In still another particular embodiment, the preservative is ozone.

Non-limiting examples of antienzymatics suitable for use as preservatives in particular embodiments of the invention include ascorbic acid, citric acid, and metal chelating agents such as ethylenediaminetetraacetic acid (EDTA).

### Hydration Agent

In certain embodiments, the functional ingredient is at least one hydration agent.

As used herein, the at least one hydration agent may be single hydration agent or a plurality of hydration agents as a functional ingredient for the compositions provided herein. Generally, according to particular embodiments of this invention, the at least one hydration agent is present in the composition in an amount sufficient to promote health and wellness.

Hydration products help the body to replace fluids that are lost through excretion. For example, fluid is lost as sweat in order to regulate body temperature, as urine in order to excrete waste substances, and as water vapor in order to exchange gases in the lungs. Fluid loss can also occur due to a wide range of external causes, non-limiting examples of which include physical activity, exposure to dry air, diarrhea, vomiting, hyperthermia, shock, blood loss, and hypotension. Diseases causing fluid loss include diabetes, cholera, gastroenteritis, shigellosis, and yellow fever. Forms of malnutrition that cause fluid loss include the excessive consumption of alcohol, electrolyte imbalance, fasting, and rapid weight loss.

In a particular embodiment, the hydration product is a composition that helps the body replace fluids that are lost during exercise. Accordingly, in a particular embodiment, the hydration product is an electrolyte, non-limiting examples of which include sodium, potassium, calcium, magnesium, chloride, phosphate, bicarbonate, and combinations thereof. Suitable electrolytes for use in particular embodiments of this invention are also described in U.S. Patent No. 5,681,569. In particular embodiments, the electrolytes are obtained from their corresponding water-soluble salts. Non-limiting examples of salts for use in particular embodiments include chlorides, carbonates, sulfates, acetates, bicarbonates, citrates, phosphates, hydrogen phosphates, tartrates, sorbates, citrates, benzoates, or combinations thereof. In other embodiments, the electrolytes are provided by juice, fruit extracts, vegetable extracts, tea, or teas extracts.

In particular embodiments of this invention, the hydration product is a carbohydrate to supplement energy stores burned by muscles. Suitable carbohydrates for use in particular embodiments of this invention are described in U.S. Patent Numbers 4,312,856, 4,853,237, 5,681,569, and 6,989,171. Non-limiting examples of suitable carbohydrates include monosaccharides, disaccharides, oligosaccharides, complex polysaccharides or combinations thereof. Non-limiting examples of suitable types of monosaccharides for use in particular embodiments include trioses, tetroses, pentoses, hexoses, heptoses, octoses, and nonoses. Non-limiting examples of specific types of suitable monosaccharides include glyceraldehyde, dihydroxyacetone, erythrose, threose, erythrulose, arabinose, lyxose, ribose, xylose, ribulose, xylulose, allose, altrose, galactose, glucose, gulose, idose, mannose, talose, fructose, psicose, sorbose, tagatose, mannoheptulose, sedoheltulose, octolose, and sialose. Non-limiting examples of suitable disaccharides include sucrose, lactose, and maltose. Non-limiting examples of suitable oligosaccharides include saccharose, maltotriose, and maltodextrin. In other particular embodiments, the carbohydrates are provided by a corn syrup, a beet sugar, a cane sugar, a juice, or a tea.

In another particular embodiment, the hydration is a flavanol that provides cellular rehydration. Flavanols are a class of natural substances present in plants, and generally comprise a 2-phenylbenzopyrone molecular skeleton attached to one or more chemical moieties. Non-limiting examples of suitable flavanols for use in particular embodiments of this invention include catechin, epicatechin, gallocatechin, epigallocatechin, epicatechin gallate, epigallocatechin 3-gallate, theaflavin, theaflavin 3-gallate, theaflavin 3'-gallate, theaflavin 3,3' gallate, thearubigin or combinations thereof. Several common sources of flavanols include tea plants, fruits, vegetables, and flowers. In preferred embodiments, the flavanol is extracted from green tea.

In a particular embodiment, the hydration product is a glycerol solution to enhance exercise endurance. The ingestion of a glycerol containing solution has been shown to provide beneficial physiological effects, such as expanded blood volume, lower heart rate, and lower rectal temperature.

### Probiotics/Prebiotics

In certain embodiments, the functional ingredient is chosen from at least one probiotic, prebiotic and combination thereof.

As used herein, the at least one probiotic or prebiotic may be single probiotic or prebiotic or a plurality of probiotics or prebiotics as a functional ingredient for the compositions provided herein. Generally, according to particular embodiments of this invention, the at least one probiotic, prebiotic or combination thereof is present in the composition in an amount sufficient to promote health and wellness.

Probiotics, in accordance with the teachings of this invention, comprise microorganisms that benefit health when consumed in an effective amount. Desirably, probiotics beneficially affect the human body's naturally-occurring gastrointestinal microflora and impart health benefits apart from nutrition. Probiotics may include, without limitation, bacteria, yeasts, and fungi.

Prebiotics, in accordance with the teachings of this invention, are compositions that promote the growth of beneficial bacteria in the intestines. Prebiotic substances can be consumed by a relevant probiotic, or otherwise assist in keeping the relevant probiotic alive or stimulate its growth. When consumed in an effective amount, prebiotics also beneficially affect the human body's naturally-occurring gastrointestinal microflora and thereby impart health benefits apart from just nutrition. Prebiotic foods enter the colon and serve as substrate for the endogenous bacteria, thereby indirectly providing the host with energy, metabolic substrates, and essential micronutrients. The body's digestion and absorption of prebiotic foods is dependent upon bacterial metabolic activity, which salvages energy for the host from nutrients that escaped digestion and absorption in the small intestine.

According to particular embodiments, the probiotic is a beneficial microorganisms that beneficially affects the human body's naturally-occurring gastrointestinal microflora and imparts health benefits apart from nutrition. Examples of probiotics include, but are not limited to, bacteria of the genus *Lactobacilli, Bifidobacteria, Streptococci,* or combinations thereof, that confer beneficial effects to humans.

In particular embodiments of the invention, the at least one probiotic is chosen from the genus *Lactobacilli. Lactobacilli* (i.e., bacteria of the genus *Lactobacillus,* hereinafter *"L."*) have been used for several hundred years as a food preservative and for promoting human health. Non-limiting examples of species of *Lactobacilli* found in the human intestinal tract include *L. acidophilus, L. casei, L. fermentum, L. saliva roes, L. brevis, L. leichmannii, L. plantarum, L. cellobiosus, L. reuteri, L. rhamnosus, L. GG, L. bulgaricus,* and *L. thermophilus,.*

According to other particular embodiments of this invention, the probiotic is chosen from the genus *Bifidobacteria. Bifidobacteria* also are known to exert a beneficial influence on human health by producing short chain fatty acids (e.g., acetic, propionic, and butyric acids), lactic, and formic acids as a result of carbohydrate metabolism. Non-limiting species of *Bifidobacteria* found in the human gastrointestinal tract include *B. angulatum, B. animalis, B. asteroides, B. bifidum, B. boum, B. breve, B. catenulatum, B. choerinum, B. coryneforme, B. cuniculi, B. dentium, B. gallicum, B. gallinarum, B indicum, B. longum, B. magnum, B. merycicum, B. minimum, B. pseudocatenulatum, B. pseudolongum, B. psychraerophilum, B. pullorum, B. ruminantium, B. saeculare, B. scardovii, B. simiae, B. subtile, B. thermacidophilum, B. thermophilum, B. urinalis, and B. sp.*

According to other particular embodiments of this invention, the probiotic is chosen from the genus *Streptococcus. Streptococcus thermophilus* is a gram-positive facultative anaerobe. It is classified as a lactic acid bacteria and commonly is found in milk and milk products, and is used in the production of yogurt. Other non-limiting probiotic species of this bacteria include *Streptococcus salivarus* and *Streptococcus cremoris.*

Probiotics that may be used in accordance with this invention are well-known to those of skill in the art. Non-limiting examples of foodstuffs comprising probiotics include yogurt, sauerkraut, kefir, kimchi, fermented vegetables, and other foodstuffs containing a microbial element that beneficially affects the host animal by improving the intestinal microbalance.

Prebiotics, in accordance with the embodiments of this invention, include, without limitation, mucopolysaccharides, oligosaccharides, polysaccharides, amino acids, vitamins, nutrient precursors, proteins and combinations thereof.

According to a particular embodiment of this invention, the prebiotic is chosen from dietary fibers, including, without limitation, polysaccharides and oligosaccharides. These compounds have the ability to increase the number of probiotics, which leads to the benefits conferred by the probiotics. Non-limiting examples of oligosaccharides that are categorized as prebiotics in accordance with particular embodiments of this invention include fructooligosaccharides, inulins, isomalto-oligosaccharides, lactilol, lactosucrose, lactulose, pyrodextrins, soy oligosaccharides, transgalacto-oligosaccharides, and xylo-oligosaccharides.

According to other particular embodiments of the invention, the prebiotic is an amino acid. Although a number of known prebiotics break down to provide carbohydrates for probiotics, some probiotics also require amino acids for nourishment.

Prebiotics are found naturally in a variety of foods including, without limitation, bananas, berries, asparagus, garlic, wheat, oats, barley (and other whole grains), flaxseed, tomatoes, Jerusalem artichoke, onions and chicory, greens (e.g., dandelion greens, spinach, collard greens, chard, kale, mustard greens, turnip greens), and legumes (e.g., lentils, kidney beans, chickpeas, navy beans, white beans, black beans).

### Weight Management Agent

In certain embodiments, the functional ingredient is at least one weight management agent.

As used herein, the at least one weight management agent may be single weight management agent or a plurality of weight management agents as a functional ingredient for the compositions provided herein. Generally, according to particular embodiments of this invention, the at least one weight management agent is present in the composition in an amount sufficient to promote health and wellness.

As used herein, "a weight management agent" includes an appetite suppressant and/or a thermogenesis agent. As used herein, the phrases "appetite suppressant", "appetite satiation compositions", "satiety agents", and "satiety ingredients" are synonymous. The phrase "appetite suppressant" describes macronutrients, herbal extracts, exogenous hormones, anorectics, anorexigenics, pharmaceutical drugs, and combinations thereof, that when delivered in an effective amount, suppress, inhibit, reduce, or otherwise curtail a person's appetite. The phrase "thermogenesis agent" describes macronutrients, herbal extracts, exogenous hormones, anorectics, anorexigenics, pharmaceutical drugs, and combinations thereof, that when delivered in an effective amount, activate or otherwise enhance a person's thermogenesis or metabolism.

Suitable weight management agents include macronutrient selected from the group consisting of proteins, carbohydrates, dietary fats, and combinations thereof. Consumption of proteins, carbohydrates, and dietary fats stimulates the release of peptides with appetite-suppressing effects. For example, consumption of proteins and dietary fats stimulates the release of the gut hormone cholecytokinin (CCK), while consumption of carbohydrates and dietary fats stimulates release of Glucagon-like peptide 1 (GLP-1).

Suitable macronutrient weight management agents also include carbohydrates. Carbohydrates generally comprise sugars, starches, cellulose and gums that the body converts into glucose for energy. Carbohydrates often are classified into two categories, digestible carbohydrates (e.g., monosaccharides, disaccharides, and starch) and non-digestible carbohydrates (e.g., dietary fiber). Studies have shown that non-digestible carbohydrates and complex polymeric carbohydrates having reduced absorption and digestibility in the small intestine stimulate physiologic responses that inhibit food intake. Accordingly, the carbohydrates embodied herein desirably comprise non-digestible carbohydrates or carbohydrates with reduced digestibility. Non-limiting examples of such carbohydrates include polydextrose; inulin; monosaccharide-derived polyols such as erythritol, mannitol, xylitol, and sorbitol; disaccharide-derived alcohols such as isomalt, lactitol, and maltitol; and hydrogenated starch hydrolysates. Carbohydrates are described in more detail herein below.

In another particular embodiment weight management agent is a dietary fat. Dietary fats are lipids comprising combinations of saturated and unsaturated fatty acids. Polyunsaturated fatty acids have been shown to have a greater satiating power than mono-unsaturated fatty acids. Accordingly, the dietary fats embodied herein desirably comprise poly-unsaturated fatty acids, non-limiting examples of which include triacylglycerols.

In a particular embodiment, the weight management agent is an herbal extract. Extracts from numerous types of plants have been identified as possessing appetite suppressant properties. Non-limiting examples of plants whose extracts have appetite suppressant properties include plants of the genus *Hoodia, Trichocaulon, Caralluma, Stapelia, Orbea, Asclepias,* and *Camelia.* Other embodiments include extracts derived from Gymnema Sylvestre, Kola Nut, Citrus Auran tium, Yerba Mate, Griffonia Simplicifolia, Guarana, myrrh, guggul Lipid, and black current seed oil.

The herbal extracts may be prepared from any type of plant material or plant biomass. Non-limiting examples of plant material and biomass include the stems, roots, leaves, dried powder obtained from the plant material, and sap or dried sap. The herbal extracts generally are prepared by extracting sap from the plant and then spray-drying the sap. Alternatively, solvent extraction procedures may be employed. Following the initial extraction, it may be desirable to further fractionate the initial extract (e.g., by column chromatography) in order to obtain an herbal extract with enhanced activity. Such techniques are well known to those of ordinary skill in the art.

In a particular embodiment, the herbal extract is derived from a plant of the genus *Hoodia,* species of which include *H. alstonii, H. currorii, H. dregei, H. flava, H. gordonii, H. jutatae, H. mossamedensis, H. officinalis, H. parviflorai, H. pedicellata, H. pilifera, H. ruschii,* and *H. triebneri. Hoodia* plants are stem succulents native to southern Africa. A sterol glycoside of *Hoodia,* known as P57, is believed to be responsible for the appetite-suppressant effect of the *Hoodia* species.

In another particular embodiment, the herbal extract is derived from a plant of the genus *Caralluma,* species of which include C. *indica, C. fimbriata, C. attenuate, C. tuberculata, C. edulis, C. adscendens, C. stalagmifera, C. umbellate, C. penicillata, C. russeliana, C. retrospicens, C. Arabica,* and C. *lasiantha. Carralluma* plants belong to the same Subfamily as *Hoodia,* Asclepiadaceae. *Caralluma* are small, erect and fleshy plants native to India having medicinal properties, such as appetite suppression, that generally are attributed to glycosides belonging to the pregnane group of glycosides, non-limiting examples of which include caratuberside A, caratuberside B, bouceroside I, bouceroside II, bouceroside III, bouceroside IV, bouceroside V, bouceroside VI, bouceroside VII, bouceroside VIII, bouceroside IX, and bouceroside X.

In another particular embodiment, the at least one herbal extract is derived from a plant of the genus *Trichocaulon. Trichocaulon* plants are succulents that generally are native to southern Africa, similar to *Hoodia,* and include the species *T. piliferum* and *T. officinale.*

In another particular embodiment, the herbal extract is derived from a plant of the genus *Stapelia* or *Orbea,* species of which include *S. gigantean* and O. *variegate,* respectively. Both *Stapelia* and *Orbea* plants belong to the same Subfamily as *Hoodia,* Asclepiadaceae. Not wishing to be bound by any theory, it is believed that the compounds exhibiting appetite suppressant activity are saponins, such as pregnane glycosides, which include stavarosides A, B, C, D, E, F, G, H, I, J, and K.

In another particular embodiment, the herbal extract is derived from a plant of the genus *Asclepias. Asclepias* plants also belong to the Asclepiadaceae family of plants. Non-limiting examples of *Asclepias* plants include *A. incarnate, A. curassayica, A. syriaca,* and *A. tuberose.* Not wishing to be bound by any theory, it is believed that the extracts comprise steroidal compounds, such as pregnane glycosides and pregnane aglycone, having appetite suppressant effects.

In a particular embodiment, the weight management agent is an exogenous hormone having a weight management effect. Non-limiting examples of such hormones include CCK, peptide YY, ghrelin, bombesin and gastrin-releasing peptide (GRP), enterostatin, apolipoprotein A-IV, GLP-1, amylin, somastatin, and leptin.

In another embodiment, the weight management agent is a pharmaceutical drug. Non-limiting examples include phentenime, diethylpropion, phendimetrazine, sibutramine, rimonabant, oxyntomodulin, floxetine hydrochloride, ephedrine, phenethylamine, or other stimulants.

### Osteoporosis Management Agent

In certain embodiments, the functional ingredient is at least one osteoporosis management agent.

As used herein, the at least one osteoporosis management agent may be single osteoporosis management agent or a plurality of osteoporosis management agent as a functional ingredient for the compositions provided herein. Generally, according to particular embodiments of this invention, the at least one osteoporosis management agent is present in the composition in an amount sufficient to promote health and wellness.

In certain embodiments, the osteoporosis management agent is at least one calcium source. According to a particular embodiment, the calcium source is any compound containing calcium, including salt complexes, solubilized species, and other forms of calcium. Non-limiting examples of calcium sources include amino acid chelated calcium, calcium carbonate, calcium oxide, calcium hydroxide, calcium sulfate, calcium chloride, calcium phosphate, calcium hydrogen phosphate, calcium dihydrogen phosphate, calcium citrate, calcium malate, calcium citrate malate, calcium gluconate, calcium tartrate, calcium lactate, solubilized species thereof, and combinations thereof.

According to a particular embodiment, the osteoporosis management agent is a magnesium soucrce. The magnesium source is any compound containing magnesium, including salt complexes, solubilized species, and other forms of magnesium. Non-limiting examples of magnesium sources include magnesium chloride, magnesium citrate, magnesium gluceptate, magnesium gluconate, magnesium lactate, magnesium hydroxide, magnesium picolate, magnesium sulfate, solubilized species thereof, and mixtures thereof. In another particular embodiment, the magnesium source comprises an amino acid chelated or creatine chelated magnesium.

In other embodiments, the osteoporosis agent is chosen from vitamins D, C, K, their precursors and/or beta-carotene and combinations thereof.

Numerous plants and plant extracts also have been identified as being effective in the prevention and treatment of osteoporosis. Not wishing to be bound by any theory, it is believed that the plants and plant extracts stimulates bone morphogenic proteins and/or inhibits bone resorption, thereby stimulating bone regeneration and strength. Non-limiting examples of suitable plants and plant extracts as osteoporosis management agents include species of the genus *Taraxacum* and *Amelanchier,* as disclosed in U.S. Patent Publication No. 2005/0106215, and species of the genus *Lindera, Artemisia, Acorus, Carthamus, Carum, Cnidium, Curcuma, Cyperus, Juniperus, Prunus, Iris, Cichorium, Dodonaea, Epimedium, Erigonoum, Soya, Mentha, Ocimum, thymus, Tanacetum, Plantago, Spearmint, Bixa, Vitis, Rosemarinus, Rhus,* and *Anethum,* as disclosed in U.S. Patent Publication No. 2005/0079232.

### Phytoestrogen

In certain embodiments, the functional ingredient is at least one phytoestrogen.

As used herein, the at least one phytoestrogen may be single phytoestrogen or a plurality of phytoestrogens as a functional ingredient for the compositions provided herein. Generally, according to particular embodiments of this invention, the at least one phytoestrogen is present in the composition in an amount sufficient to promote health and wellness.

Phytoestrogens are compounds found in plants which can typically be delivered into human bodies by ingestion of the plants or the plant parts having the phytoestrogens. As used herein, "phytoestrogen" refers to any substance which, when introduced into a body causes an estrogen-like effect of any degree. For example, a phytoestrogen may bind to estrogen receptors within the body and have a small estrogen-like effect.

Examples of suitable phytoestrogens for embodiments of this invention include, but are not limited to, isoflavones, stilbenes, lignans, resorcyclic acid lactones, coumestans, coumestroI, equol, and combinations thereof. Sources of suitable phytoestrogens include, but are not limited to, whole grains, cereals, fibers, fruits, vegetables, black cohosh, agave root, black currant, black haw, chasteberries, cramp bark, dong quai root, devil's club root, false unicorn root, ginseng root, groundsel herb, licorice, liferoot herb, motherwort herb, peony root, raspberry leaves, rose family plants, sage leaves, sarsaparilla root, saw palmetto berried, wild yam root, yarrow blossoms, legumes, soybeans, soy products (e.g., miso, soy flour, soymilk, soy nuts, soy protein isolate, tempen, or tofu) chick peas, nuts, lentils, seeds, clover, red clover, dandelion leaves, dandelion roots, fenugreek seeds, green tea, hops, red wine, flaxseed, garlic, onions, linseed, borage, butterfly weed, caraway, chaste tree, vitex, dates, dill, fennel seed, gotu kola, milk thistle, pennyroyal, pomegranates, southernwood, soya flour, tansy, and root of the kudzu vine (pueraria root) and the like, and combinations thereof.

Isoflavones belong to the group of phytonutrients called polyphenols. In general, polyphenols (also known as "polyphenolics"), are a group of chemical substances found in plants, characterized by the presence of more than one phenol group per molecule.

Suitable phytoestrogen isoflavones in accordance with embodiments of this invention include genistein, daidzein, glycitein, biochanin A, formononetin, their respective naturally occurring glycosides and glycoside conjugates, matairesinol, secoisolariciresinol, enterolactone, enterodiol, textured vegetable protein, and combinations thereof.

Suitable sources of isoflavones for embodiments of this invention include, but are not limited to, soy beans, soy products, legumes, alfalfa sprouts, chickpeas, peanuts, and red clover.

### Long-Chain Primary Aliphatic Saturated Alcohol

In certain embodiments, the functional ingredient is at least one long chain primary aliphatic saturated alcohol.

As used herein, the at least one long chain primary aliphatic saturated alcohol may be single long chain primary aliphatic saturated alcohol or a plurality of long chain primary aliphatic saturated alcohols as a functional ingredient for the compositions provided herein. Generally, according to particular embodiments of this invention, the at least one long chain primary aliphatic saturated alcohol is present in the composition in an amount sufficient to promote health and wellness.

Long-chain primary aliphatic saturated alcohols are a diverse group of organic compounds. The term alcohol refers to the fact these compounds feature a hydroxyl group (-OH) bound to a carbon atom. The term primary refers to the fact that in these compounds the carbon atom which is bound to the hydroxyl group is bound to only one other carbon atom. The term saturated refers to the fact that these compounds feature no carbon to carbon pi bonds. The term aliphatic refers to the fact that the carbon atoms in these compounds are joined together in straight or branched chains rather than in rings. The term long-chain refers to the fact that the number of carbon atoms in these compounds is at least 8 carbons).

Non-limiting examples of particular long-chain primary aliphatic saturated alcohols for use in particular embodiments of the invention include the 8 carbon atom 1-octanol, the 9 carbon 1-nonanol, the 10 carbon atom 1-decanol, the 12 carbon atom 1-dodecanol, the 14 carbon atom 1-tetradecanol, the 16 carbon atom 1-hexadecanol, the 18 carbon atom 1-octadecanol, the 20 carbon atom 1-eicosanol, the 22 carbon 1-docosanol, the 24 carbon 1-tetracosanol, the 26 carbon 1-hexacosanol, the 27 carbon 1-heptacosanol, the 28 carbon 1-octanosol, the 29 carbon 1-nonacosanol, the 30 carbon 1-triacontanol, the 32 carbon 1-dotriacontanol, and the 34 carbon 1-tetracontanol.

In a particularly desirable embodiment of the invention, the long-chain primary aliphatic saturated alcohols are policosanol. Policosanol is the term for a mixture of long-chain primary aliphatic saturated alcohols composed primarily of 28 carbon 1-octanosol and 30 carbon 1-triacontanol, as well as other alcohols in lower concentrations such as 22 carbon 1-docosanol, 24 carbon 1-tetracosanol, 26 carbon 1-hexacosanol, 27 carbon 1-heptacosanol, 29 carbon 1-nonacosanol, 32 carbon 1-dotriacontanol, and 34 carbon 1-tetracontanol.

Long-chain primary aliphatic saturated alcohols are derived from natural fats and oils. They may be obtained from these sources by using extraction techniques well known to those of ordinary skill in the art. Policosanols can be isolated from a variety of plants and materials including sugar cane *(Saccharum officinarium),* yams *(e.g. Dioscorea opposite),* bran from rice *(e.g. Oryza sativa),* and beeswax. Policosanols may be obtained from these sources by using extraction techniques well known to those of ordinary skill in the art. A description of such extraction techniques can be found in U.S. Pat. Appl. No. 2005/0220868*.*

### Phytosterols

In certain embodiments, the functional ingredient is at least one phytosterol, phytostanol or combination thereof.

Generally, according to particular embodiments of this invention, the at least one phytosterol, phytostanol or combination thereof is present in the composition in an amount sufficient to promote health and wellness.

As used herein, the phrases "stanol", "plant stanol" and "phytostanol" are synonymous.

Plant sterols and stanols are present naturally in small quantities in many fruits, vegetables, nuts, seeds, cereals, legumes, vegetable oils, bark of the trees and other plant sources. Although people normally consume plant sterols and stanols every day, the amounts consumed are insufficient to have significant cholesterol-lowering effects or other health benefits. Accordingly, it would be desirable to supplement food and beverages with plant sterols and stanols.

Sterols are a subgroup of steroids with a hydroxyl group at C-3. Generally, phytosterols have a double bond within the steroid nucleus, like cholesterol; however, phytosterols also may comprise a substituted side chain (R) at C-24, such as an ethyl or methyl group, or an additional double bond. The structures of phytosterols are well known to those of skill in the art.

At least 44 naturally-occurring phytosterols have been discovered, and generally are derived from plants, such as corn, soy, wheat, and wood oils; however, they also may be produced synthetically to form compositions identical to those in nature or having properties similar to those of naturally-occurring phytosterols. According to particular embodiments of this invention, non-limiting examples of phytosterols well known to those or ordinary skill in the art include 4-desmethylsterols (e.g., β-sitosterol, campesterol, stigmasterol, brassicasterol, 22-dehydrobrassicasterol, and Δ5-avenasterol), 4-monomethyl sterols, and 4,4-dimethyl sterols (triterpene alcohols) (e.g., cycloartenol, 24-methylenecycloartanol, and cyclobranol).

As used herein, the phrases "stanol", "plant stanol" and "phytostanol" are synonymous. Phytostanols are saturated sterol alcohols present in only trace amounts in nature and also may be synthetically produced, such as by hydrogenation of phytosterols. According to particular embodiments of this invention, non-limiting examples of phytostanols include β-sitostanol, campestanol, cycloartanol, and saturated forms of other triterpene alcohols.

Both phytosterols and phytostanols, as used herein, include the various isomers such as the α and β isomers (e.g., α-sitosterol and β-sitostanol, which comprise one of the most effective phytosterols and phytostanols, respectively, for lowering serum cholesterol in mammals).

The phytosterols and phytostanols of the present invention also may be in their ester form. Suitable methods for deriving the esters of phytosterols and phytostanols are well known to those of ordinary skill in the art, and are disclosed in U.S. Patent Numbers 6,589,588, 6,635,774, 6,800,317, and U.S. Patent Publication Number 2003/0045473. Non-limiting examples of suitable phytosterol and phytostanol esters include sitosterol acetate, sitosterol oleate, stigmasterol oleate, and their corresponding phytostanol esters. The phytosterols and phytostanols of the present invention also may include their derivatives.

Generally, the amount of functional ingredient in the composition varies widely depending on the particular composition and the desired functional ingredient. Those of ordinary skill in the art will readily ascertain the appropriate amount of functional ingredient for each composition.

### VI. Methods

Methods of enhancing the sweetness of a consumable and/or modulating one or more taste attributes of the sweetener to make the consumable taste more like a sucrose-sweetened consumable are described but not claimed.

In one embodiment, a method of enhancing the sweetness of a consumable comprises (i) providing a consumable comprising at least one sweetener described hereinabove and (ii) adding at least one dihydrochalcone of the formulae described herein to the consumable to provide a consumable with enhanced sweetness.

In another embodiment, a method of enhancing the sweetness of a consumable comprises (i) providing a consumable matrix and (ii) adding at least one sweetener described hereinabove and at least one dihydrochalcone of the formulae described herein to the consumable matrix to provide a consumable with enhanced sweetness. The at least one sweetener and at least one dihydrochalcone can be added together, i.e. in the form of a composition, or separately. Optionally, the at least one sweetener can be added in the form of a sweetener component.

As used herein, the term "consumable matrix" refers to a consumable containing all typical ingredients except the sweetener or sweetener component.

In a particular embodiment, the SE of the consumable comprising the at least one dihydrochalcone and at least one sweetener is enhanced by at least about 1.2-fold compared to the SE of the consumable in the absence of the at least one dihydrochalcone, such as, for example, at least about 1.3-fold, at least about 1.4-fold, at least about 1.5-fold, at least about 1.6-fold, at least about 1.7-fold, at least about 1.8-fold, at least about 1.9-fold and at least about 2.0-fold.

In another embodiment, addition of the at least one dihydrochalcone to the consumable or consumable matrix increases the degrees Brix by at least 1 degree Brix compared to the Brix of the consumable without the dihydrochalcone, such as, for example, at least 2 degrees Brix, at least 3 degrees Brix or at least 4 degrees Brix.

In a particular embodiment, the consumable is a beverage.

Accordingly, a method of enhancing the sweetness of a beverage comprises (i) providing a beverage comprising at least one sweetener described hereinabove and (ii) adding at least one dihydrochalcone of the formulae described herein to the beverage to provide a beverage with enhanced sweetness.

In another embodiment, a method of enhancing the sweetness of a beverage comprises (i) providing a beverage matrix and (ii) adding at least one sweetener described hereinabove and at least one dihydrochalcone of the formulae described herein to the beverage matrix to provide a beverage with enhanced sweetness. The at least one sweetener and at least one dihydrochalcone can be added together, i.e. in the form of a composition, or separately. Optionally, the at least one sweetener can be added in the form of a sweetener component.

In another aspect, a method of making a consumable taste more like a sucrose-sweetened consumable comprises (i) providing a consumable comprising at least one sweetener described hereinabove and (ii) adding at least one dihydrochalcone of the formulae described herein in an amount effective to modulate one or more taste attributes of the sweetener to make the consumable taste more like a sucrose-sweetened consumable compared to the consumable in the absence of the at least one dihydrochalcone.

In another embodiment, a method of making a consumable taste more like a sucrose-sweetened consumable comprises (i) providing a consumable matrix and (ii) adding at least one sweetener described hereinabove and at least one dihydrochalcone of the formulae described herein to the consumable to provide a consumable that tastes more like a sucrose-sweetened consumable, wherein the at least one dihydrochalcone is present in an amount effective to modulate one or more taste attributes of the sweetener to make the consumable taste more like a sucrose-sweetened consumable compared to the consumable in the absence of the at least one dihydrochalcone. The at least one sweetener and at least one dihydrochalcone can be added together, i.e. in the form of a composition, or separately. Optionally, the at least one sweetener can be added in the form of a sweetener component.

As discussed above, in the present methods the at least one dihydrochalcone may be present in the consumable in the identified concentrations. Methods or preparing consumables with enhanced sweetness are also provided.

In one aspect, a method of preparing a consumable comprises (i) providing a consumable comprising at least one sweetener described hereinabove and (ii) adding at least one dihydrochalcone of the formulae described herein to the consumable to provide a consumable.

In another aspect, a method of preparing a consumable comprises (i) providing a consumable matrix and (ii) adding at least one sweetener described hereinabove and at least one dihydrochalcone of the formulae described herein to the consumable matrix to provide a consumable. The at least one sweetener and at least one dihydrochalcone can be added together, i.e. in the form of a composition, or separately. Optionally, the at least one sweetener can be added in the form of a sweetener component.

In still another aspect, a method of preparing a consumable comprises (i) providing a consumable matrix and (ii) adding a composition comprising at least one sweetener described hereinabove and at least one dihydrochalcone of the formulae described herein to the consumable matrix to provide a consumable. Optionally, the at least one sweetener can be added in the form of a sweetener component.

In yet another aspect, a method of preparing a beverage comprises (i) providing a beverage matrix and (ii) adding at least one sweetener described hereinabove and at least one dihydrochalcone of the formulae described herein to the beverage matrix to provide a beverage. The at least one sweetener and at least one dihydrochalcone can be added together, i.e. in the form of a composition, or separately. Optionally, the at least one sweetener can be added in the form of a sweetener component.

In a further aspect, a method of preparing a beverage comprises (i) providing a beverage matrix and (ii) adding a composition comprising at least one sweetener described hereinabove and at least one dihydrochalcone of the formulae described herein to the beverage matrix to provide a beverage. Optionally, the at least one sweetener can be added in the form of a sweetener component.

In a still further aspect, a method of preparing a sweetened beverage comprises (i) providing an unsweetened beverage and (ii) adding at least one sweetener described hereinabove and at least one dihydrochalcone of the formulae described herein to the unsweetened beverage to provide a sweetened beverage. The at least one sweetener and at least one dihydrochalcone can be added together, i.e. in the form of a composition, or separately. Optionally, the at least one sweetener can be added in the form of a sweetener component.

In another aspect, a method of preparing a sweetened beverage comprises (i) providing an unsweetened beverage and (ii) adding a composition comprising at least one sweetener described hereinabove and at least one dihydrochalcone of the formulae described herein to the unsweetened beverage to provide a sweetened beverage. Optionally, the at least one sweetener can be added in the form of a sweetener component.

### EXAMPLES

### EXAMPLE 1: Sweetness Enhancement of Rebaudioside M with Phloretin (Reference)

The ability of phloretin to impact the sweetness profile of rebaudioside M was evaluated.

### Sample Preparation

Samples were prepared with the following ingredients:

**Table 1: 40 ppm of phloretin in acidic water**

| **Ingredient** | **Amount** |
|---|---|
| Phloretin | 10 mg |
| Propylene glycol | 0.5 g |
| Citric acid | 62.5 mg |
| Water | 249.5 g |

Initially, phloretin is dissolved in propylene glycol. Then, water and citric acid were added. Propylene glycol is used to improve the solubility of phloretin.

**Table 2: Acidic water**

| **Ingredient** | **Amount** |
|---|---|
| Propylene glycol | 0.6 g |
| Citric acid | 75 mg |
| Water | 299.4 g |

**Table 3: 150 ppm of Rebaudioside M in acidic water**

| **Ingredient** | **Amount** |
|---|---|
| Rebaudioside M | 15 mg |
| Acidic water (Table 2) | 100 g |

**Table 4: 40 ppm of phloretin and 150 ppm of Rebaudioside M in acidic water**

| **Ingredient** | **Amount** |
|---|---|
| Rebaudioside M | 15 mg |
| Phloretin in acidic water (Table 1) | 100 g |

**Table 5: Sucrose in acidic water**

| **Ingredient** | **1.5% Sucrose** | **5% Sucrose** | **7% Sucrose** | **8% Sucrose** | **9% Sucrose** | **10% Sucrose** |
|---|---|---|---|---|---|---|
| Sucrose | 1.5 g | 5 g | 7 g | 8 g | 9 g | 10 g |
| Citric acid | 25 mg | 25 mg | 25 mg | 25 mg | 25 mg | 25 mg |
| Water | 98.5 g | 95 g | 93 g | 92 g | 91 g | 90 g |

Ingredients were added to water or water with phloretin while stirring until solids were visibly dissolved and the sample was poured into a glass bottle and stored at 4°C.

### Taste Evaluation

Taste tests were carried out with one panelist. Bottles were removed from the refrigerator and about 25 ml of beverage was poured into 4 oz- plastic cups. The panelist was given mineral water to rinse their mouth before tasting and between tasting different samples. Unsalted crackers were also given to eat followed by rinsing their mouth with mineral water before tasting the next sample.

First, the panelist assessed the sweetness of 40 ppm of phloretin in acidic water against a 1.5% sucrose solution in acidic water. 40 ppm of phloretin was reported to have a sweetness less than the sweetness of the 1.5% sucrose solution.

Next, the panelist compared the sweetness profile of (i) the sample containing 150 ppm rebaudioside M in acidic water and (ii) the sample containing 40 ppm phloretin and 150 ppm rebaudioside M in acidic water to the 5%, 7%, 8%, 9%, and 10% sucrose solutions. The panelist was instructed to sip, evaluate the sweetness, and then spit the samples in cups provided for that purpose.

The sample containing 150 ppm rebaudioside M was equally sweet to the 6% sucrose solution. The sample containing 40 ppm phloretin and 150 ppm Rebaudioside M was equally sweet to the 9% sucrose solution and had more mouthfeel.

### EXAMPLE 2: Sweetness Enhancement of Rebaudioside M with Neohesperidin Dihydrochalcone (Reference)

The ability of neohesperidin dihydrochalcone (NHDC) to impact the sweetness profile of rebaudioside M was evaluated.

### Sample Preparation

Sample were prepared with the following ingredients:

**Table 1: 5 ppm of NHDC in acidic water**

| **Ingredient** | **Amount** |
|---|---|
| NHDC | 2.5 mg |
| Citric acid | 125 mg |
| Water | 500 g |

**Table 2: acidic water**

| **Ingredient** | **Amount** |
|---|---|
| Citric acid | 125 mg |
| Water | 500 g |

**Table 3: 5 ppm of NHDC and 150 ppm of Rebaudioside M in acidic water**

| **Ingredient** | **Amount** |
|---|---|
| Rebaudioside M | 30 mg |
| NHDC in acidic Water (Table 1) | 200 g |

**Table 4: 150 ppm of Rebaudioside M in acidic water**

| **Ingredient** | **Amount** |
|---|---|
| Rebaudioside M | 30 mg |
| Acidic Water (Table 2) | 200 g |

**Table 5: Sucrose in acidic water**

| **Ingredient** | **1.5% Sucrose** | **6% Sucrose** | **8% Sucrose** | **10% Sucrose** |
|---|---|---|---|---|
| Water | 98.5 g | 94 g | 92 g | 90 g |
| Sucrose | 1.5 g | 6 g | 8 g | 10 g |
| Citric acid | 25 mg | 25 mg | 25 mg | 25 mg |

Ingredients were added to water or water with NHDC while stirring until solids were visibly dissolved and the sample was poured into a glass bottle and stored at 4°C.

### Taste Evaluation

Taste tests were carried out with two panelists. Bottles were removed from the refrigerator and about 25 ml of the sample was poured into 4 oz- plastic cups. Panelists were given mineral water to rinse their mouth before tasting and between tasting different samples. Unsalted crackers were also given to panelists to eat followed by rinsing their mouth with mineral water before tasting the next sample.

First, the panelists assessed the sweetness of 5 ppm of NHDC in acidic water against 1.5% sucrose solution in acidic water. Both panelists found that 5 ppm of NHDC had less sweetness than the 1.5% sucrose solution.

Next, panelists compared the sweetness profile of (i) a sample containing 150 ppm rebaudioside M and (ii) a sample containing 5 ppm NHDC and 150 rebaudioside M to the 6%, 8%, and 10% sucrose solutions. Panelists were instructed to sip, evaluate the sweetness, and then spit the samples in cups provided for that purpose.

Both panelists agreed that the sample containing 150 ppm rebaudioside M was equally sweet to the 6% sucrose solution, and that the sample containing 5 ppm NHDC and 150 ppm rebaudioside M was equally sweet to the 8% sucrose solution. One panelist reported that the sample containing 5 ppm NHDC and 150 ppm rebaudioside M had a quick sweet on-set. One panelist reported that the sample containing 5 ppm NHDC and 150 ppm rebaudioside M exhibited sugar-like sweetness.

### EXAMPLE 3: Sweetness Enhancement of Rebaudioside M with Hesperetin Dihydrochalcone

The ability of hesperetin dihydrochalcone to impact the sweetness profile of rebaudioside M was evaluated.

### Sample Preparation

Samples were prepared with the following ingredients:

**Table 1: 40 ppm of Hesperetin dihydrochalcone in acidic water**

| **Ingredient** | **Amount** |
|---|---|
| Hesperetin dihydrochalcone | 6 mg |
| Propylene glycol | 0.3 g |
| Citric acid | 37.5 mg |
| Water | 150 g |

| | |
|---|---|
| * Initially Hesperetin dihydrochalcone is dissolved in Propylene glycol. Then, water and citric acid were added. Propylene glycol is used to improve the solubility of Hesperetin dihydrochalcone. | |

**Table 2: Acidic water**

| **Ingredient** | **Amount** |
|---|---|
| Propylene glycol | 0.6 g |
| Citric acid | 75 mg |
| Water | 299.4 g |

**Table 3: 150 ppm of Rebaudioside M in acidic water**

| **Ingredient** | **Amount** |
|---|---|
| Rebaudioside M | 15 mg |
| Acidic water (Table 2) | 100 g |

**Table 4: 40 ppm of Hesperetin dihydrochalcone and 150 ppm of Rebaudioside M in acidic water**

| **Ingredient** | **Amount** |
|---|---|
| Rebaudioside M | 11.3 mg |
| Hesperetin dihydrochalcone in acidic water (Table 1) | 75 g |

**Table 5: Sucrose in acidic water**

| **Ingredient** | **4% Sucrose** | **6% Sucrose** | **8% Sucrose** | **10% Sucrose** |
|---|---|---|---|---|
| Sucrose | 4 g | 6 g | 8 g | 10 g |
| Citric acid | 25 mg | 25 mg | 25 mg | 25 mg |
| Water | 96 g | 94 g | 92 g | 90 g |

Ingredients were added to water or water with Hesperetin dihydrochalcone while stirring until solids were visibly dissolved and the sample was poured into a glass bottle and stored at 4°C.

### Taste Evaluation

Taste tests were carried out with two panelists. Bottles were removed from the refrigerator and about 25 ml of beverage was poured into 4 oz- plastic cups. Panelists were given mineral water to rinse their mouth before tasting and between tasting different samples. Unsalted crackers were also given to panelists to eat followed by rinsing their mouth with mineral water before tasting the next sample.

Panelists compared the sweetness profile of (i) a sample containing 150 ppm rebaudioside M and (ii) a sample containing 40 ppm hesperetin dihydrochalcone and 150 ppm rebaudioside M to the 4%, 6%, 8%, and 10% sucrose solutions. Panelists were instructed to sip, evaluate the sweetness, and then spit the samples in cups provided for that purpose.

Both panelists agreed that the sample containing 150 ppm rebaudioside M was slightly less sweet than the 6% sucrose solution. One panelist found that the sample containing 40 ppm hesperetin dihydrochalcone and 150 ppm rebaudioside M was sweeter than 9% sucrose, was lightly bitter, and had a heavier mouthfeel. The other panelist found that the sample containing 40 ppm hesperetin dihydrochalcone and 150 ppm rebaudioside M was sweeter than the 10% solution and had better mouthfeel than the sample containing only 150 ppm rebaudioside M. Both panelists assessed that the sample containing hesperetin dihydrochalcone and rebaudioside M was significantly sweeter than the sample containing only rebaudioside M, and that the sample containing hesperetin dihydrochalcone had more mouthfeel.

### EXAMPLE 4: Sweetness Enhancement of Siamenoside I with Hesperetin Dihydrochalcone

The ability of hesperetin dihydrochalcone to impact the sweetness profile of rebaudioside M was evaluated.

### Sample Preparation

Sample were prepared with the following ingredients:

**Table 1: 40 ppm of Hesperetin dihydrochalcone in acidic water**

| **Ingredient** | **Amount** |
|---|---|
| Hesperetin dihydrochalcone | 6 mg |
| Propylene glycol | 0.3 g |
| Citric acid | 37.5 mg |
| Water | 150 g |

| | |
|---|---|
| * Initially hesperetin dihydrochalcone is dissolved in Propylene glycol. Then, water and citric acid were added. Propylene glycol is used to improve the solubility of hesperetin dihydrochalcone. | |

**Table 2: Acidic water**

| **Ingredient** | **Amount** |
|---|---|
| Propylene glycol | 0.6 g |
| Citric acid | 75 mg |
| Water | 299.4 g |

**Table 3: 150 ppm of Siamenoside I in acidic water**

| **Ingredient** | **Amount** |
|---|---|
| Siamenoside I | 15 mg |
| Acidic water (Table 2) | 100 g |

**Table 4: 40 ppm of hesperetin dihydrochalcone and 150 ppm of Siamenoside I in acidic water**

| **Ingredient** | **Amount** |
|---|---|
| Siamenoside I | 11.3 mg |
| Hesperetin dihydrochalcone in acidic water (Table 1) | 75 g |

**Table 5: Sucrose in acidic water**

| **Ingredient** | **4% Sucrose** | **6% Sucrose** | **8% Sucrose** | **10% Sucrose** |
|---|---|---|---|---|
| Sucrose | 4 g | 6 g | 8 g | 10 g |
| Citric acid | 25 mg | 25 mg | 25 mg | 25 mg |
| Water | 96 g | 94 g | 92 g | 90 g |

Ingredients were added to water or water with hesperetin dihydrochalcone while stirring until solids were visibly dissolved and the sample was poured into a glass bottle and stored at 4°C.

### Taste Evaluation

Taste tests were carried out with two panelists. Bottles were removed from the refrigerator and about 25 ml of beverage was poured into 4 oz- plastic cups. Panelists were given mineral water to rinse their mouth before tasting and between tasting different samples. Unsalted crackers were also given to panelists to eat followed by rinsing their mouth with mineral water before tasting the next sample.

Panelists compared the sweetness profile of (i) a sample containing 150 ppm siamenoside I and (ii) a sample containing 40 ppm hesperetin dihydrochalcone and 150 ppm siamenoside I to the 4%, 6%, 8%, and 10% sucrose solutions. Panelists were instructed to sip, evaluate the sweetness, and then spit the samples in cups provided for that purpose.

Both panelists agreed that the sample containing 150 ppm siamenoside I was slightly less sweet than the 6% sucrose solution. One panelist reported that the sample containing 40 ppm hesperetin dihydrochalcone and 150 ppm siamenoside I was sweeter than 9% sucrose in acidic water and had a heavier mouthfeel. The other panelist found that the sample containing hesperetin dihydrochalcone and siamenoside I was sweeter than the 10% sucrose solution and had better mouthfeel than the sample containing 150 ppm siamenoside I. Both panelists assessed that the sample containing hesperetin dihydrochalcone and siamenoside I was significantly sweeter than the sample containing only siamenoside I, and that the sample containing hesperetin dihydrochalcone had more mouthfeel.

### EXAMPLE 5: Hesperetin Dihydrochalcone Enhancement of Rebaudioside A and Rebaudioside M

### Material and Methods

Rebaudioside M was acquired from Pure Circle (86% Reb-M, 9% Reb-D, 0.8% Reb-A, plus other steviol glycosides in minor amount).

A 6% stock solution of Hesperetin Dihydrochalcone (CC-00800) was made in propylene glycol (PG) food grade and used in subsequent experiments.

### Beverage Preparation

### 1. Mock Beverages

Acidified mock beverages (300 grams) were made using the following ingredients (in grams) in order:

| **Ingredients** | **Bev. #1** | **Bev. #2** | **Bev. #3** | **Bev. #4** | **Bev. #5** | **Bev. #6** | **Bev. #7** |
|---|---|---|---|---|---|---|---|
| | 15 ppm CC-00800 + 300 ppm Reb-M | 20 ppm CC-00800 + 300 ppm Reb-M | 30 ppm CC-00800 + 300 ppm Reb-M | 35 ppm CC-00800 + 315 ppm Reb-M | 10 ppm CC-00800 + 350 ppm Reb-M | 20 ppm CC-00800 + 320 ppm Reb-M | Control 472 ppm Reb-M |
| Water | 299.4 | 299.38 | 299.33 | 299.3 | 299.41 | 299.37 | 299.32 |
| Citric acid | 0.351 | 0.351 | 0.351 | 0.351 | 0.351 | 0.351 | 0.351 |
| Sodium citrate | 0.0825 | 0.0825 | 0.0825 | 0.0825 | 0.0825 | 0.0825 | 0.0825 |
| Reb-M | 0.09 | 0.09 | 0.09 | 0.095 | 0.105 | 0.096 | 0.142 |
| CC-00800 in PG | 0.075 | 0.1 | 0.15 | 0.175 | 0.05 | 0.1 | 0.1 * |
| Total | 300 g | 300 g | 300 g | 300 g | 300 g | 300 g | 300 g |

| | | | | | | | |
|---|---|---|---|---|---|---|---|
| * PG spiked in beverage | | | | | | | |

All ingredients were dissolved in carbon-filtered water and the resulting beverage was filled in 300 ml glass bottles and immediately refrigerated (4°C). Beverages were bench tasted next morning.

### 2. Diet Carbonated Lemon Lime

The following ingredients were used to make 1 liter of beverage:

| Ingredients | **Bev. #1** | **Bev. # 2** |
|---|---|---|
| | 15 ppm CC-00800 + 320 ppm Reb-M | Control Reb-M 472 ppm |
| Water | 996.85 | 996.77 |
| Citric acid | 1.171 | 1.171 |
| Sodium citrate | 0.275 | 0.275 |
| Sodium benzoate | 0.185 | 0.185 |
| Lemon lime flavor | 0.865 | 0.865 |
| Reb-M | 0.320 | 0.472 |
| CC-00800 in PG | 0.250 | 0.250* |
| Total | 1000 g | 1000 g |

| | | |
|---|---|---|
| * PG spiked in beverage | | |

The diet lemon lime beverages were carbonated with beverage grade carbon dioxide to a carbonation level of 3.8 volume, then filled in 300 ml glass bottles and aged over night at 35°C. Next day the beverages were cooled at 4°C then bench tasted.

### 3. Diet Berry Hydration Water

The following ingredients were used to make 300 g of finished beverage:

| Ingredients | **Bev. #1** | **Bev. #2** | **Bev. #3** | **Bev. # 4** |
|---|---|---|---|---|
| | 10 ppm CC-00800 + 200 ppm Reb-A | 15 ppm CC-00800 + 200 ppm Reb-A | 20 ppm CC-00800 + 200 ppm Reb-A | Control Reb-A 200 ppm |
| Water | 297.54 | 297.5 | 297.49 | 297.5 |
| Citric acid | 0.229 | 0.229 | 0.229 | 0.229 |
| Ascorbic acid | 0.15 | 0.15 | 0.15 | 0.15 |
| Vitamin premix | 0.088 | 0.088 | 0.088 | 0.088 |
| Mineral premix | 0.001 | 0.001 | 0.001 | 0.001 |
| Natural color | 0.208 | 0.208 | 0.208 | 0.208 |
| Berry flavor | 0.174 | 0.174 | 0.174 | 0.174 |
| Reb-A | 0.06 | 0.06 | 0.06 | 0.06 |
| CC-00800 in PG | 0.05 | 0.075 | 0.1 | 0.075* |
| Erythritol | 1.5 | 1.5 | 1.5 | 1.5 |
| Total | 300 g | 300 g | 300 g | 300 g |

| | | | | |
|---|---|---|---|---|
| * PG spiked in beverage | | | | |

All ingredients were dissolved in carbon-filtered water. After dissolution the beverages were filled in 400 ml PET bottles, stored at 4°C overnight, and bench tasted next morning.

### Bench Tasting and Results

Five experienced panelists bench tasted the beverages blindly. Each panelist was given warm bottled water and unsalted crackers to eat and rinse the palate between samples. A maximum of 3 samples was tasted at each session to avoid fatigue.

### 1. Mock Beverages

All panelists agreed that Beverages #1 to 6 tasted sweeter than the control with 472 ppm of Reb-M. Also, their sweetness profile was much improved with no sweet lingering, licorice and bitter aftertaste observed in the control. The sweetness onset was fast with a sugar-like profile and more mouthfeel.

### 2. Diet Carbonated Lemon Lime

All panelists agreed that Beverage #1 was similar in overall sweetness to the control with 472 ppm of Reb-M. However, beverage #1 had more mouthfeel with significant reduction of sweet lingering and licorice aftertaste.

### 3. Diet Berry Hydration Water

All panelists agreed that Beverages #1 to 3 were sweeter than the control, with more mouthfeel and clean profile without sweet lingering.

### Conclusion

CC-00800 improved beverages with either Reb-M or Reb-A in terms of sweetness qualities and flavor profile. The beverages with less amount of steviol glycoside were found to be much sweeter than their controls, which indicates enhancement by CC-00800.

### EXAMPLE 6: Hesperetin Dihydrochalcone Enhancement of Rebaudiosides J, N, O and M

### Material and Methods

Steviol glycosides, namely Reb-J, Reb-N, Reb-M, Reb-O with 95% purity were purchased from commercial sources.

A 6% stock solution of **CC-00800** (hesperitin dihydrochalcone, "HDC") was made in propylene glycol (PG) food grade and used in subsequent experiments.

### Beverage Preparation

### 1. Mock Beverages

### A. Mock Beverages with Steviol Glycosides (200 ppm)

Acidified mock beverages (100 grams) were made using the following ingredients (in grams) in order:

| **Ingredients** | **Bev. #1** | **Bev. #2** | **Bev. #3** | **Bev. #4** | **Bev. #5** | **Bev. #6** | **Bev. #7** | **Bev. #8** |
|---|---|---|---|---|---|---|---|---|
| | 200 ppm Reb-J | 10 ppm CC-00800 + 200 ppm Reb-J | 200 ppm Reb-N | 10 ppm CC-00800 + 200 ppm Reb-N | 200 ppm Reb-O | 10 ppm CC-00800 + 200 ppm Reb-O | 200 ppm Reb-M | 10 ppm CC-00800 + 200 ppm Reb-M |
| Water | 99.84 | 99.82 | 99.84 | 99.82 | 99.84 | 99.82 | 99.84 | 99.82 |
| Citric acid | 0.117 | 0.117 | 0.117 | 0.117 | 0.117 | 0.117 | 0.117 | 0.117 |
| Sodium citrate | 0.027 | 0.027 | 0.027 | 0.027 | 0.027 | 0.027 | 0.027 | 0.027 |
| Steviol Glycoside | 0.02 | 0.02 | 0.02 | 0.02 | 0.02 | 0.02 | 0.02 | 0.02 |
| CC-00800 in PG | - | 0.017 | - | 0.017 | - | 0.017 | - | 0.017 |
| Total | 100 g | 100 g | 100 g | 100g | 100 g | 100 g | 100 g | 100 g |

### B. Mock Beverages with Steviol Glycosides (400 ppm)

| **Ingredients** | **Bev. #9** | **Bev. #10** | **Bev. #11** | **Bev. #12** | **Bev. #13** | **Bev. #14** |
|---|---|---|---|---|---|---|
| | 400 ppm Reb-J | 10 ppm CC-00800 + 400 ppm Reb-J | 400 ppm Reb-N | 10 ppm CC-00800 + 400 ppm Reb-N | 400 ppm Reb-M | 10 ppm CC-00800 + 400 ppm Reb-M |
| Water | 99.82 | 99.80 | 99.82 | 99.80 | 99.82 | 99.80 |
| Citric acid | 0.117 | 0.117 | 0.117 | 0.117 | 0.117 | 0.117 |
| Sodium citrate | 0.027 | 0.027 | 0.027 | 0.027 | 0.027 | 0.027 |
| Reb-M | 0.04 | 0.04 | 0.04 | 0.04 | 0.04 | 0.04 |
| CC-00800 in PG | - | 0.017 | - | 0.017 | - | 0.017 |
| Total | 100 g | 100 g | 100 g | 100 g | 100 g | 100 g |

### C. Mock Beverages with Sucrose (References)

| **Ingredients** | **Ref. #1** | **Ref. #2** | **Ref. #3** |
|---|---|---|---|
| | 7 Brix Sucrose | 8 Brix Sucrose | 10 Brix Sucrose |
| Water | 92.86 | 91.86 | 89.86 |
| Citric acid | 0.117 | 0.117 | 0.117 |
| Sodium citrate | 0.027 | 0.027 | 0.027 |
| Sucrose granular | 7 | 8 | 10 |
| Total | 100 | 100 | 100 |

All ingredients were dissolved in carbon-filtered water and the resulting beverage was filled in 300 ml glass bottles and immediately refrigerated (4°C). Beverages were bench tasted when cold.

### Sensory Tasting and Results

Four experienced panelists bench tasted the beverages blindly. Each panelist was given warm bottled water and unsalted crackers to eat and rinse the palate between samples. A maximum of 3 samples was tasted at each session to avoid fatigue.

### 1. Mock Beverages

The first session consisted of tasting beverages without hesperitin dihydrochalcone. The results are shown in the table below. Reb-M was the sweetest, while Reb-J was the least sweet and most bitter.

| **Beverage** | **Steviol Glycoside (ppm)** | **Sweetness Equivalent (Brix sucrose)** | **Panelist Comments** |
|---|---|---|---|
| #1 (Reb-J) | 200 | 6-7 | Least sweet, most bitter, sour |
| #9 (Reb-J) | 400 | 7-8 | Still bitter, increased sweetness |
| #3 (Reb-N) | 200 | 6-7 | Slight bitterness at the end |
| #11 (Reb-N) | 400 | 8-9 | Clean and rounded sweetness |
| #5 (Reb-O) | 200 | 6-7 | Less bitter than Reb-J at same level |
| #7 (Reb-M) | 200 | 7-8 | Nice and clean sweetness |
| #13 (Reb-M) | 400 | 9 | Most sweet, clean, fast sweet onset, more rounded |

The second session consisted of tasting the beverages with hesperitin dihydrochalcone added (10 ppm). The results are shown in the table below. All panelists agreed that hesperitin dihydrochalcone enhanced the overall sweetness for about 1 Brix for Reb-J, Reb-O, and Reb-N, while Reb-M was enhanced with about 2.5 Brix. Hesperitin dihydrochalcone also improved the mouth feel and reduced significantly the bitterness and other off-notes, making the beverages more pleasant in the mouth.

| **Beverage** | **Steviol Glycoside (ppm)** | **CC-00800 (ppm)** | **Sweetness Equivalent (Brix sucrose)** | **Panelist Comments** |
|---|---|---|---|---|
| #2 (Reb-J) | 200 | 10 | 7 | Improved mouthfeel, reduced bitterness |
| #10 (Reb-J) | 400 | 10 | 8-9 | More sweetness with much improved mouthfeel, no bitterness |
| #4 (Reb-N) | 200 | 10 | 7-8 | No bitterness, good mouthfeel |
| #12 (Reb-N) | 400 | 10 | 10 | Improved mouth feel, no bitterness, improved sweetness |
| #6 (Reb-O) | 200 | 10 | 7-8 | Creamy notes, improved mouth feel |
| #8 (Reb-M) | 200 | 10 | 10 | Good mouth feel, sugar-like taste |
| #14 (Reb-M) | 400 | 10 | 12 | Fast sweet onset, pleasant mouth feel, well balanced sweetness |

### EXAMPLE 7: ENHANCEMENT OF 1,6-α ISOMER OF SIAMENOSIDE I WITH HESPERITIN DIHYDROCHALCONE

The following 1,6-α isomer of Siamenoside I (mogro-3-O-[β-D-glucopyranoside]-24-O-{[β-D-glucopyranosyl-(1→2)]-[α-D-glucopyranosyl-(1→6)]-β-D-glucopyranoside}) was prepared using the method described in US Patent Application Publication No. 2017/0119032 by converting Mogroside IIIe with cyclomaltodextrin glucanotransferase in the presence of starch:

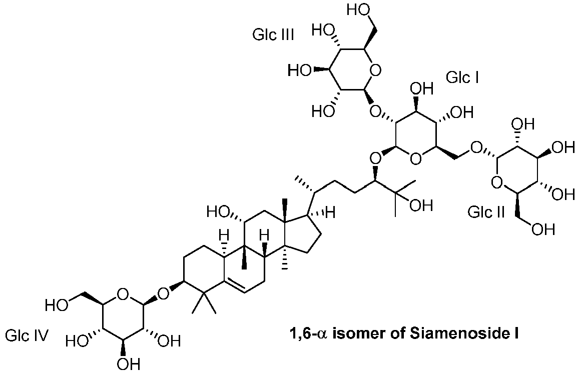

A mixture of 0.5 g of Mogroside IIIe, 2.5 g of soluble starch, and 15 mL of CGTase (Toruzyme 3.0L) from Novozyme in 50 mL of pH 5 sodium acetate buffer was stirred at 50 °C for 24 h. The reaction was stopped by heating at 80 °C, cooled to room temperature and then centrifuged at 3600 rpm for 10 minutes to remove the unreacted enzyme.

Primary Prep-HPLC Processing: The supernatant was injected in its entirety, approximately 60 mL, and processed using the conditions listed in the table below.

| **Parameters** | |
|---|---|
| Column (Dimensions) | Phenomenex Synergi Hydro RP (50 × 250 mm, 7 µm ) |
| Column Temperature (°C) | Ambient |
| Sample Temperature (°C) | Ambient |
| Mobile Phases | 77:23 Water/MeCN (A) 10:90 Water/MeCN (B) |
| Flow Rate (mL/min) | 118.0 |

| **Gradient** | |
|---|---|
| Isocratic hold of 100% MPA for 30 min | |

Three target peaks between 14 and 22 minutes were collected as fractions.

Secondary Prep-HPLC Processing: Fraction Pool from primary processing was processed using the conditions listed below:

| **Parameters** | |
|---|---|
| Column (Dimensions) | Waters SymmetryShield RP18 (50 × 250 mm, 7 µm ) |
| Column Temperature (°C) | Ambient |
| Sample Temperature (°C) | Ambient |
| Mobile Phases | 78:22 Water/MeCN (A) 10:90 |
| | Water/MeCN (B) |
| Flow Rate (mL/min) | 118.0 |

| **Gradient** | |
|---|---|
| Isocratic hold of 100% MP-A for 40 min, then 7 min flush of 100% MP-B | |

Peaks were collected as fractions. Analytical HPLC analysis was performed on the fractions. The peak containing the 1,6-α isomer of Siamenoside I had a retention time of 32.4 mins. 19.8 mg of material was obtained.

Structural elucidation was carried out with 3.2 mg dissolved in 150 µL CD₃OD. The following NMR spectra were obtained: ¹H, ¹³C, ¹H-¹H COSY, ¹H-¹³C HSQC-DEPT and ¹H-¹³C HMBC. The spectra indicated the presence of a central triterpene core and four sugar units in the structure. Analysis of available NMR data indicated the presence of four glucose units in the structure. Of the four anomeric protons, three were observed in the ¹H NMR spectrum acquired at 300K and all had large coupling constant (7.5-7.8 Hz), indicating that they had β-configurations. The remaining anomeric proton obscured by water resonance in the ¹H NMR spectrum acquired at 300K was resolved in the spectrum acquired at 296K and had a small coupling (3.6 Hz), indicating that this had α-configuration. The linkages between the glucose units and their attachment to the central triterpene core were determined based on ¹H-¹H COSY, 1D-TOCSY, ¹H-¹³C HSQC-DEPT and ¹H-¹³C HMBC data acquired at 300K. The key ¹H-¹H COSY and ¹H-¹³C HMBC correlations utilized to confirm the sugar linkages. The ¹H NMR spectra of the compound is provided in Figure 1.

### Preparation of Hesperetin Dihydrochalcone

Three batches of hesperetin dihydrochalcone were prepared using neohesperidin dihydrochalcone (NHDC) as starting material. In total, 162.23 g of hesperetin dihydrochalcone was prepared.

The first batch was prepared by selective hydrolysis of NHDC using diluted hydrochloric acid (1.5 M HCl) in EtOH (1:1, v/v) at 85 °C over 15h. Our goal was to prepare both hesperetin dihydrochalcone and hesperetin dihydrochalcone 4'-b-D-glucoside in a single reaction run. During hydrolysis, the reaction mixture turned intense red.

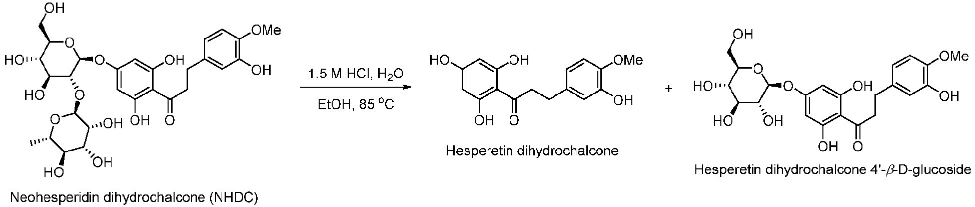

After the hydrolysis was completed, the EtOH was removed under reduced pressure and the products were separated by selective extraction. Hesperetin dihydrochalcone was selectively extracted from the aqueous phase with a mixture of Et₂O and hexane (1:1, v/v), while hesperetin dihydrochalcone 4'-*β*-D-glucoside was extracted with EtOAc. Each product was purified separately by flash column chromatography on silica gel. Hesperetin dihydrochalcone was further purified by crystallization/precipitation twice from EtOH and water (1:2, v/v) at room temperature. Hesperetin dihydrochalcone 4'-*β*-D-glucoside was purified by crystallization from EtOH and then by preparative HPLC.

The second batch of hesperetin dihydrochalcone was prepared by complete hydrolysis of NHDC using 3.0 M hydrochloric acid in EtOH (1:1, v/v) at 95 °C over 5h. In total, 217.83 g of NHDC was hydrolyzed in four separated batches (50.33 g, 51.00 g, 51.00 g and 65.50 g). The reaction mixture turned intense red during the hydrolysis.

After the hydrolysis was complete, the EtOH was removed under reduced pressure and the product was extracted with a mixture of Et₂O and hexane (3:1, v/v). Each batch was purified separately by flash column chromatography on silica gel. The combined product was further purified by crystallization/precipitation twice from EtOH and water (1:2, v/v) at room temperature. The second batch provided 66.93 g (62%) of hesperetin dihydrochalcone (>99% purity based on LCMS).

The third batch of hesperetin dihydrochalcone was prepared by complete hydrolysis of two batches of NHDC under different conditions. The first batch of NHDC (152.50 g) was hydrolyzed using 2.0 M hydrochloric acid in EtOH (1:1, v/v) at 85 °C over 24h under a nitrogen atmosphere. The second batch of NHDC (200.50 g) was hydrolyzed using 2.5 M hydrochloric acid in EtOH (1:1, v/v) at 85 °C over 8h under a nitrogen atmosphere. The intense red coloration of the reaction mixtures was observed.

After the hydrolysis was complete, the EtOH was removed under reduced pressure to provide a solid precipitate. The solid was collected by filtration and dried, which gave crude hesperetin dihydrochalcone. The crude material was purified by flash column chromatography on silica gel. The combined product was further purified by crystallization/precipitation twice from EtOH and water (1:2, v/v) at room temperature. The third batch provided 87.42 g (50%) of hesperetin dihydrochalcone (>99.9% purity based on LCMS).

### Enhancement of 1,6-α Isomer of Siamenoside with Hesperitin Dihydrochalcone

Sample were prepared with the following ingredients:

**Table 1: 40 ppm of Hespertin dihydrochalcone in acidic water**

| **Ingredient** | **Amount** |
|---|---|
| Hespertin dihydrochalcone | 10 mg |
| Propylene glycol | 0.5 g |
| Citric acid | 62.5 mg |
| Water | 249.5 g |

**Table 2: Acidic water**

| **Ingredient** | **Amount** |
|---|---|
| Propylene glycol | 0.5 g |
| Citric acid | 62.5 mg |
| Water | 249.5 g |

**Table 3: 200 ppm of Isomer of Siamenoside I in acidic water**

| **Ingredient** | **Amount** |
|---|---|
| Isomer of Siamenoside I | 1 mg |
| Acidic water (Table 2) | 5 g |

**Table 4: 100 ppm of Isomer of Siamenoside I in acidic water**

| **Ingredient** | **Amount** |
|---|---|
| 200 ppm of Isomer of Siamenoside I in acidic water (Table 3) | 2.5 g |
| Acidic water (Table 2) | 2.5 g |

**Table 5: 20 ppm of Hespertin dihydrochalcone and 100 ppm of Isomer of Siamenoside I in acidic water**

| **Ingredient** | **Amount** |
|---|---|
| 200 ppm of Isomer of Siamenoside I in acidic water | 2.5 g |
| 40 ppm of Hespertin dihydrochalcone in acidic water (Table 1) | 2.5 g |

**Table 6: Sucrose in acidic water**

| **Ingredient** | **3% Sucrose** | **5% Sucrose** | **8% Sucrose** |
|---|---|---|---|
| Sucrose | 3 g | 5 g | 8 g |
| Citric acid | 25 mg | 25 mg | 25 mg |
| Water | 97 g | 95 g | 92 g |

The samples were stored at 4°C. Taste tests were carried out with one panelist. The samples were removed from the refrigerator and about 5 ml of beverage was poured into 1 oz-plastic cups. Panelist was given mineral water to rinse their mouth before tasting and between tasting different samples. Unsalted crackers were also given to panelist to eat followed by rinsing the mouth with mineral water before tasting the next sample.

The sucrose solutions, a sample of Isomer of Siamenoside I in acidic water and a sample of Hespertin dihydrochalcone and Isomer of Siamenoside I in acidic water were given to the panelist. The panelist was asked to evaluate the sweetness of the samples against the 3%, 5%, and 8% sucrose solutions and describe the taste profiles. The panelist was instructed to sip, evaluate the sweetness, and then spit the samples in cups provided for that purpose.

The panelist assessed that the sample containing the Isomer of Siamenoside I was found to be about as sweet as 4% sucrose in acidic water and the sample containing Hespertin dihydrochalcone and Isomer of Siamenoside I was about as sweet as 8% sucrose in acidic water. The panelist determined that the sample containing Hespertin dihydrochalcone and Isomer of Siamenoside I was significantly sweeter than the sample containing Isomer of Siamenoside I and had slightly more mouthfeel.

### EXAMPLE 8: ENHANCEMENT OF REBAUDIOSIDE M WITH HESPERETIN DIHYDROCHALCONE-4'-β-D-GLUCOSIDE

Sample were prepared with the following ingredients:

**Table 1: 2% of Hesperetin dihydrochalcone-4'-beta-D- glucoside in PG**

| **Ingredient** | **Amount** |
|---|---|
| Hesperetin dihydrochalcone-4'-beta-D-glucoside | 50 mg |
| Propylene glycol | 2.5 g |

**Table 2: 250 ppm of Rebaudioside M in acidic water**

| **Ingredient** | **Amount** |
|---|---|
| Rebaudioside M | 125 mg |
| Water | 499.75 g |
| Citric acid | 125 mg |

**Table 3: 20 ppm of Hesperetin dihydrochalcone-4'-beta-D-glucoside and 250 ppm of Rebaudioside M in acidic water**

| **Ingredient** | **Amount** |
|---|---|
| 250 ppm of Rebaudioside M in acidic water (Table 2) | 200 g |
| Hesperetin dihydrochalcone-4'-beta-D-glucoside in PG (Table 1) | 0.2 g |

**Table 4: 250 ppm of Rebaudioside M and PG in acidic water (Control)**

| **Ingredient** | **Amount** |
|---|---|
| 250 ppm of Rebaudioside M in acidic water (Table 2) | 200 g |
| PG | 0.2 g |

Hesperetin dihydrochalcone-4'-beta-D-glucoside in PG was measured in weighing boat and added to the Rebaudioside M solution while stirring. The weighing boat was rinsed with Rebaudioside M solution. The solution was stirred visibly clear and the sample was poured into a glass bottle and stored at 4°C.

Taste tests were carried out with two panelists. Bottles were removed from the refrigerator and about 25 ml of beverage was poured into 4 oz- plastic cups. Panelists were given mineral water to rinse their mouth before tasting and between tasting different samples. Unsalted crackers were also given to panelists to eat followed by rinsing their mouth with mineral water before tasting the next sample.

A sample of Rebaudioside M in acidic water and a sample of Hesperetin dihydrochalcone-4'-beta-D-glucoside and Rebaudioside M in acidic water were given to panelists. Panelists were asked to evaluate the sweetness of samples and describe the difference of taste profile. Panelists were instructed to sip, evaluate the sweetness, and then spit the samples in cups provided for that purpose.

Both panelists assessed the sample containing Rebaudioside M was found to be less sweet and less mouthfeel than the sample containing Hesperetin dihydrochalcone-4'-beta-D-glucoside and Rebaudioside M. One panelist assessed that the sweetness of 250 ppm of Rebaudioside M in acidic water was slightly less sweet than 7% sucrose and the sweetness of 20 ppm of Hesperetin dihydrochalcone-4'-beta-D-glucoside and 250 ppm of Rebaudioside M in acidic water was slightly less sweet than 10% sucrose. 20 ppm of Hesperetin dihydrochalcone-4'-beta-D-glucoside and 250 ppm of Rebaudioside M in acidic water was found to have more mouthfeel than 250 ppm of Rebaudioside M in acidic water. The other panelist assessed that the sweetness of 250 ppm of Rebaudioside M in acidic water was slightly less sweet than 8% sucrose and the sweetness of 20 ppm of Hesperetin dihydrochalcone-4'-beta-D-glucoside and 250 ppm of Rebaudioside M in acidic water was equivalent to 9% sucrose equivalent sweetness. 20 ppm of Hesperetin dihydrochalcone-4'-beta-D-glucoside and 250 ppm of Rebaudioside M in acidic water was found to have more mouthfeel than 250 ppm of Rebaudioside M in acidic water.

### EXAMPLE 9: ENHANCEMENT OF SIAMENOSIDE I WITH HESPERETIN DIHYDROCHALCONE-4'-β-D-GLUCOSIDE

Samples were prepared with the following ingredients:

**Table 1: 2% of Hesperetin dihydrochalcone-4'-beta-D-glucoside in PG**

| **Ingredient** | **Amount** |
|---|---|
| Hesperetin dihydrochalcone-4'-beta-D-glucoside | 50 mg |
| Propylene glycol | 2.5 g |

**Table 2: 250 ppm of Siamenoside I in acidic water**

| **Ingredient** | **Amount** |
|---|---|
| Siamenoside I | 50 mg |
| Water | 200 g |
| Citric acid | 50 mg |

**Table 3: 20 ppm of Hesperetin dihydrochalcone-4'-beta-D-glucoside and 250 ppm of Siamenoside I in acidic water**

| **Ingredient** | **Amount** |
|---|---|
| 250 ppm of Siamenoside I in acidic water (Table 2) | 100 g |
| Hesperetin dihydrochalcone-4'-beta-D-glucoside in PG (Table 1) | 0.1 g |

**Table 4: 250 ppm of Siamenoside I and PG in acidic water (Control)**

| **Ingredient** | **Amount** |
|---|---|
| 250 ppm of Siamenoside I in acidic water (Table 2) | 100 g |
| PG | 1.2 g |

Hesperetin dihydrochalcone-4'-β -D-glucoside in propylene glycol was measured in a weighing boat and added to the Siamenoside I solution while stirring. The weighing boat was rinsed with Siamenoside I solution. The solution was stirred until visibly clear and the sample was poured into a glass bottle and stored at 4°C.

Taste tests were carried out with two panelists. Bottles were removed from the refrigerator and about 25 ml of beverage was poured into 4 oz- plastic cups. Panelists were given mineral water to rinse their mouth before tasting and between tasting different samples. Unsalted crackers were also given to panelists to eat followed by rinsing their mouth with mineral water before tasting the next sample.

A sample of Siamenoside I in acidic water and a sample of hesperetin dihydrochalcone-4'-β-D-glucoside and Siamenoside I in acidic water were given to panelists. Panelists were asked to evaluate the sweetness of samples and describe the difference of taste profile. Panelists were instructed to sip, evaluate the sweetness, and then spit the samples in cups provided for that purpose.

Both panelists assessed the sample containing Siamenoside I was found to be less sweet and have less mouthfeel than the sample containing hesperetin dihydrochalcone-4'-β-D-glucoside and Siamenoside I. One panelist assessed that the sweetness of 250 ppm of Siamenoside I in acidic water was close to 6% sucrose and the sweetness of 20 ppm of hesperetin dihydrochalcone-4'-β-D-glucoside and 250 ppm of Siamenoside I in acidic water was close to 8% sucrose. 20 ppm of hesperetin dihydrochalcone-4'-β-D-glucoside and 250 ppm of Siamenoside I in acidic water was found to have slightly more mouthfeel than 250 ppm of Siamenoside I in acidic water. The other panelist assessed that the sweetness of 250 ppm of Siamenoside I in acidic water was less sweet (about 1-2 sucrose equivalent sweetness) than the sweetness of 20 ppm of hesperetin dihydrochalcone-4'-β-D-glucoside and 250 ppm of Siamenoside I in acidic water. 20 ppm of hesperetin dihydrochalcone-4'-β-D-glucoside and 250 ppm of Siamenoside I in acidic water was found to have slightly more mouthfeel than 250 ppm of siamenoside I in acidic water.

### EXAMPLE 10: ENHANCEMENT OF THE 1,6-α ISOMER OF SIAMENOSIDE I WITH HESPERETIN DIHYDROCHALCONE-4'-β-D-GLUCOSIDE

Samples were prepared with the following ingredients:

**Table 1: 2% of Hesperetin dihydrochalcone-4'-beta-D- glucoside in PG**

| **Ingredient** | **Amount** |
|---|---|
| Hesperetin dihydrochalcone-4'-beta-D-glucoside | 50 mg |
| Propylene glycol | 2.5 g |

**Table 2: 250 ppm of 1,6-α-isomer of Siamenoside I in acidic water**

| **Ingredient** | **Amount** |
|---|---|
| 1,6-α-isomer of Siamenoside I | 25 mg |
| Water | 100 g |
| Citric acid | 25 mg |

**Table 3: 20 ppm of Hesperetin dihydrochalcone-4'-beta-D-glucoside and 250 ppm of 1,6-α-isomer of Siamenoside I in acidic water**

| **Ingredient** | **Amount** |
|---|---|
| 250 ppm of 1,6-α-isomer of Siamenoside I in acidic water (Table 2) | 50 g |
| Hesperetin dihydrochalcone-4'-beta-D-glucoside in PG (Table 1) | 0.05 g |

**Table 4: 250 ppm of 1,6-α-isomer of Siamenoside I and PG in acidic water (Control)**

| **Ingredient** | **Amount** |
|---|---|
| 250 ppm of 1,6-α-isomer of Siamenoside I in acidic water (Table 2) | 50 g |
| PG | 0.05 g |

Hesperetin dihydrochalcone-4'-β-D-glucoside in PG was measured in a weighing boat and added to 1,6-α-isomer of Siamenoside I solution while stirring. The weighing boat is rinsed with 1,6-α-isomer of Siamenoside I solution. The solution was stirred visibly clear and the sample was poured into a glass bottle and stored at 4°C.

Taste tests were carried out with two panelists. Bottles were removed from the refrigerator and about 25 ml of beverage was poured into 4 oz- plastic cups. Panelists were given mineral water to rinse their mouth before tasting and between tasting different samples. Unsalted crackers were also given to panelists to eat followed by rinsing their mouth with mineral water before tasting the next sample.

A sample of 1,6-α-isomer of Siamenoside I in acidic water and a sample of hesperetin dihydrochalcone-4'-β-D-glucoside and 1,6-α-isomer of Siamenoside I in acidic water were given to panelists. Panelists were asked to evaluate the sweetness of samples and describe the difference of taste profile. Panelists were instructed to sip, evaluate the sweetness, and then spit the samples in cups provided for that purpose.

Both panelists assessed the sample containing 1,6-α-isomer of Siamenoside I was found to be less sweet and have less mouthfeel than the sample containing hesperetin dihydrochalcone-4'-β-D-glucoside and 1,6-α-isomer of Siamenoside I. One panelist assessed that the sweetness of 250 ppm of 1,6-α-isomer of Siamenoside I in acidic water was slightly less than 7% sucrose and the sweetness of 20 ppm of hesperetin dihydrochalcone-4'-β-D-glucoside and 250 ppm of 1,6-α-isomer of Siamenoside I in acidic water was slightly less than 9% sucrose. 20 ppm of hesperetin dihydrochalcone-4'-β-D-glucoside and 250 ppm of 1,6-α-isomer of Siamenoside I in acidic water was found to have slightly more mouthfeel than 250 ppm of 1,6-α-isomer of Siamenoside I in acidic water. The other panelist assessed that 250 ppm of 1,6-α-isomer of Siamenoside I in acidic water was less sweet (about 1-2 sucrose equivalent sweetness) than the sweetness of 20 ppm of hesperetin dihydrochalcone-4'-β-D-glucoside and 250 ppm of 1,6-α-isomer of Siamenoside I in acidic water. 20 ppm of hesperetin dihydrochalcone-4'-β-D-glucoside and 250 ppm of 1,6-α-isomer of Siamenoside I in acidic water was found to have slightly more mouthfeel than 250 ppm of 1,6-α-isomer of Siamenoside I in acidic water.

## Claims

1. A beverage comprising at least one sweetener and at least one dihydrochalcone, wherein the dihydrochalcone is selected from hesperetin dihydrochalcone and hesperetin dihydrochalcone-4'-β-D-glucoside, and
wherein the sweetener comprises Rebaudioside M in a sweetening amount or at least one of siamenoside I and the 1,6-α isomer of siamenoside I in a sweetening amount.

2. The beverage of claim 1, wherein the dihydrochalcone is hesperetin dihydrochalcone and the sweetener comprises rebaudioside M.

3. The beverage of claim 2, wherein the concentration of hesperetin dihydrochalcone is from about 1 ppm to about 50 ppm and the concentration of the sweetener is from about 100 ppm to about 600 ppm.

4. The beverage of claim 2, wherein the concentration of hesperetin dihydrochalcone is from about 10 ppm to about 35 ppm and the concentration of sweetener is from about 250 ppm to about 350 ppm.

5. The beverage of claim 1, wherein the dihydrochalcone is hesperetin dihydrochalcone and the sweetener comprises siamenoside I.

6. The beverage of claim 1, wherein the dihydrochalcone is hesperetin dihydrochalcone and the sweetener comprises the 1,6-α isomer of siamenoside I.

7. The beverage of claim 1, wherein the dihydrochalcone is hesperetin dihydrochalcone-4'-β-D-glucoside and the sweetener comprises rebaudioside M.

8. The beverage of claim 1, wherein the dihydrochalcone is hesperetin dihydrochalcone-4'-β-D-glucoside and the sweetener comprises siamenoside I.

9. The beverage of claim 1, wherein the dihydrochalcone is hesperetin dihydrochalcone-4'-β-D-glucoside and the sweetener comprises the 1,6-α isomer of siamenoside I.

10. The beverage of any one of claims 5-9, wherein the sucrose equivalence of the beverage is enhanced by at least about 1.2-fold compared to the sucrose equivalence of a beverage without the at least one dihydrochalcone.

11. The beverage of claim 10, wherein at least one taste attribute of the beverage is improved compared to the at least one taste attribute of a beverage without the at least one dihydrochalcone.

12. The beverage of any one of claims 1-11, wherein the beverage is a low- or zero-calorie beverage, preferably a zero-calorie beverage.

13. The beverage of any one of claims 1-12, wherein the beverage is selected from the group consisting of a frozen carbonated beverage, enhanced sparkling beverage, cola, fruit-flavored sparkling beverage, ginger-ale, soft drink, root beer, fruit juice, fruit-flavored juice, juice drink, nectar, vegetable juice, vegetable-flavored juice, sports drink, energy drink, enhanced water drink, enhanced water with vitamins, near water drink, coconut water, tea type drink, coffee, cocoa drink, beverage containing milk components, beverage containing cereal extracts and a smoothie.

14. The beverage of claim 13, wherein the beverage is a low- or zero-calorie fruit-flavored sparkling beverage or wherein the beverage is an enhanced water beverage.

15. The beverage of claim 13, wherein the beverage comprises water, natural cola flavor, a colorant comprising caramel, an acidulant, and caffeine.

## Patentansprüche

1. Getränk, das mindestens einen Süßstoff und mindestens ein Dihydrochalcon umfasst, wobei das Dihydrochalcon aus Hesperetin-Dihydrochalcon und Hesperetin-Dihydrochalcon-4'-β-D-glucosid ausgewählt ist, und
wobei der Süßstoff Rebaudiosid M in einer süßenden Menge oder Siamenosid I und/oder das 1, 6-α-Isomer von Siamenosid I in einer süßenden Menge umfasst.

2. Getränk nach Anspruch 1, wobei das Dihydrochalcon Hesperetin-Dihydrochalcon ist und der Süßstoff Rebaudiosid M umfasst.

3. Getränk nach Anspruch 2, wobei die Konzentration an Hesperetin-Dihydrochalcon etwa 1 ppm bis etwa 50 ppm beträgt und die Konzentration des Süßstoffs etwa 100 ppm bis etwa 600 ppm beträgt.

4. Getränk nach Anspruch 2, wobei die Konzentration an Hesperetin-Dihydrochalcon etwa 10 ppm bis etwa 35 ppm beträgt und die Konzentration des Süßstoffs etwa 250 ppm bis etwa 350 ppm beträgt.

5. Getränk nach Anspruch 1, wobei das Dihydrochalcon Hesperetin-Dihydrochalcon ist und der Süßstoffs Siamenosid I umfasst.

6. Getränk nach Anspruch 1, wobei das Dihydrochalcon Hesperetin-Dihydrochalcon ist und der Süßstoff das 1,6-α-Isomer von Siamenosid I umfasst.

7. Getränk nach Anspruch 1, wobei das Dihydrochalcon Hesperetin-Dihydrochalcon-4'-β-D-glucosid ist und der Süßstoff Rebaudiosid M umfasst.

8. Getränk nach Anspruch 1, wobei das Dihydrochalcon Hesperetin-Dihydrochalcon-4'-β-D-glucosid ist und der Süßstoff Siamenosid I umfasst.

9. Getränk nach Anspruch 1, wobei das Dihydrochalcon Hesperetin-Dihydrochalcon-4'-β-D-glucosid ist und der Süßstoff das 1,6-α-Isomer von Siamenosid I umfasst.

10. Getränk nach einem der Ansprüche 5-9, wobei die Saccharoseäquivalenz des Getränks im Vergleich zu der Saccharoseäquivalenz eines Getränks ohne das mindestens eine Dihydrochalcon um mindestens etwa das 1,2-fache erhöht ist.

11. Getränk nach Anspruch 10, wobei mindestens ein Geschmacksattribut des Getränks im Vergleich zu dem mindestens einen Geschmacksattribut eines Getränks ohne das mindestens eine Dihydrochalcon verbessert ist.

12. Getränk nach einem der Ansprüche 1-11, wobei das Getränk ein kalorienarmes oder kalorienfreies Getränk, vorzugsweise ein kalorienfreies Getränk, ist.

13. Getränk nach einem der Ansprüche 1-12, wobei das Getränk aus der aus einem gefrorenen kohlensäurehaltigen Getränk, einem verbesserten Schaumgetränk, Cola, einem Schaumgetränk mit Fruchtgeschmack, einem Ingwerbier, einem alkoholfreiem Getränk, einem Wurzelbier, einem Fruchtsaft, einem Saft mit Fruchtgeschmack, einem Saftgetränk, einem Nektar, einem Gemüsesaft, einem Saft mit Gemüsegeschmack, einem Sportgetränk, einem Energiegetränk, einem verbesserten Wassergetränk, einem verbesserten Wasser mit Vitaminen, Near Water Drink, Kokoswasser, einem Teegetränk, Kaffee, einem Kakaogetränk, einem Milchbestandteile enthaltendem Getränk, einem Getreideextrakt enthaltendem Getränk und einem Smoothie bestehenden Gruppe ausgewählt ist.

14. Getränk nach Anspruch 13, wobei das Getränk ein kalorienarmes oder kalorienfreies Schaumgetränk mit Fruchtgeschmack ist oder wobei das Getränk ein verbessertes Wassergetränk ist.

15. Getränk nach Anspruch 13, wobei das Getränk Wasser, natürliches Cola-Aroma, einen Farbstoff, der Karamell umfasst, ein Säuerungsmittel und Coffein umfasst.

## Revendications

1. Boisson comprenant au moins un édulcorant et au moins une dihydrochalcone, la dihydrochalcone étant choisie parmi l'hespérétine dihydrochalcone et l'hespérétine dihydrochalcone-4'-β-D-glucoside, et l'édulcorant comprenant du rébaudioside M en quantité édulcorante ou au moins l'un parmi le siaménoside I et l'isomère 1,6-α de siaménoside I en quantité édulcorante.

2. Boisson selon la revendication 1, dans laquelle la dihydrochalcone est l'hespérétine dihydrochalcone et l'édulcorant comprend du rébaudioside M.

3. Boisson selon la revendication 2, dans laquelle la concentration en hespérétine dihydrochalcone est d'environ 1 ppm à environ 50 ppm et la concentration en édulcorant est d'environ 100 ppm à environ 600 ppm.

4. Boisson selon la revendication 2, dans laquelle la concentration en hespérétine dihydrochalcone est d'environ 10 ppm à environ 35 ppm et la concentration en édulcorant est d'environ 250 ppm à environ 350 ppm.

5. Boisson selon la revendication 1, dans laquelle la dihydrochalcone est l'hespérétine dihydrochalcone et l'édulcorant comprend du siaménoside I.

6. Boisson selon la revendication 1, dans laquelle la dihydrochalcone est l'hespérétine dihydrochalcone et l'édulcorant comprend l'isomère 1,6-α de siaménoside I.

7. Boisson selon la revendication 1, dans laquelle la dihydrochalcone est l'hespérétine dihydrochalcone-4'-β-D-glucoside et l'édulcorant comprend du rébaudioside M.

8. Boisson selon la revendication 1, dans laquelle la dihydrochalcone est l'hespérétine dihydrochalcone-4'-β-D-glucoside et l'édulcorant comprend du siaménoside I.

9. Boisson selon la revendication 1, dans laquelle la dihydrochalcone est l'hespérétine dihydrochalcone-4'-β-D-glucoside et l'édulcorant comprend l'isomère 1,6-α de siaménoside I.

10. Boisson selon l'une quelconque des revendications 5 à 9, dans laquelle l'équivalence en saccharose est augmentée d'au moins 1,2 fois par rapport à l'équivalence en saccharose d'une boisson sans la ou les dihydrochalcones.

11. Boisson selon la revendication 10, dans laquelle au moins un attribut gustatif de la boisson est amélioré par rapport à au moins un attribut gustatif d'une boisson sans dihydrochalcone.

12. Boisson selon l'une quelconque des revendications 1 à 11, la boisson étant une boisson à faible teneur en calories ou sans calories, de préférence une boisson sans calories.

13. Boisson selon l'une quelconque des revendications 1 à 12, la boisson étant choisie dans le groupe constitué par une boisson gazeuse glacée, une boisson pétillante enrichie, un cola, une boisson pétillante aromatisée aux fruits, un soda au gingembre, une boisson sans alcool, un soda racinette, un jus de fruits, un jus aromatisé aux fruits, une boisson à base de jus, un nectar, un jus de légumes, un jus aromatisé aux légumes, une boisson pour sportifs, une boisson énergisante, une boisson à base d'eau enrichie, une eau enrichie en vitamines, une boisson à base d'eau, l'eau de coco, une boisson de type thé, le café, une boisson au cacao, une boisson contenant des composants laitiers, une boisson contenant des extraits de céréales et une boisson fouettée.

14. Boisson selon la revendication 13, la boisson étant une boisson pétillante aromatisée aux fruits à faible teneur en calories ou sans calories, ou la boisson étant une boisson à base d'eau enrichie.

15. Boisson selon la revendication 13, la boisson comprenant de l'eau, un arôme naturel de cola, un colorant comprenant du caramel, un acidulant et de la caféine.
